# EUROPEAN PATENT SPECIFICATION

(11) **EP 2 431 445 B1**
(45) Date of publication and mention of the grant of the patent: **23.11.2016**
(21) Application number: 10775132.3
(22) Date of filing: 14.05.2010
(51) Int. Cl.: C09K 11/06, C09B 57/00, H01L 51/00, H01L 51/50, H05B 33/14

(54) **COMPOUND FOR ORGANIC PHOTOELECTRIC DEVICE AND ORGANIC PHOTOELECTRIC DEVICE COMPRISING SAME**
VERBINDUNG FÜR ORGANISCHE PHOTOELEKTRISCHE VORRICHTUNG UND ORGANISCHE PHOTOELEKTRISCHE VORRICHTUNG DAMIT
COMPOSÉ POUR DISPOSITIF PHOTOÉLECTRIQUE ORGANIQUE ET DISPOSITIF PHOTOÉLECTRIQUE ORGANIQUE COMPRENANT CE COMPOSÉ

(30) Priority: 15.05.2009 KR 20090042706
(43) Date of publication of application: 21.03.2012
(73) Proprietor: CHEIL INDUSTRIES INC., Kumi-city Kyungsangbuk-do 730-030 (KR)
(72) Inventor: KIM, Hyung-Sun, Uiwang-si Gyeonggi-do 437-711 (KR); LEE, Ho-Jae, Uiwang-si Gyeonggi-do 437-711 (KR); KANG, Eui-Su, Uiwang-si Gyeonggi-do 437-711 (KR); LEE, Nam-Heon, Uiwang-si Gyeonggi-do 437-711 (KR); PARK, Young-Sung, Uiwang-si Gyeonggi-do 437-711 (KR); YU, Eun-Sun, Uiwang-si Gyeonggi-do 437-711 (KR); CHAE, Mi-Young, Uiwang-si Gyeonggi-do 437-711 (KR)
(74) Representative: Michalski Hüttermann & Partner Patentanwälte mbB
(86) International application number: PCT/KR2010/003071
(87) International publication number: WO 2010/131930

(56) References cited:
- EP-A1- 1 400 578
- EP-A1- 1 690 847
- EP-A1- 1 752 440
- JP-A- 2005 085 599
- KR-A- 20080 012 015
- US-A- 5 698 740
- US-A1- 2002 182 439
- US-A1- 2007 051 944
- US-A1- 2007 141 390
- US-A1- 2007 141 390
- US-A1- 2008 036 365

## Description

### [Technical Field]

A compound for an organic photoelectric device and an organic photoelectric device including the same are related.

### [Background Art]

An organic photoelectric device is, in a broad sense, a device for transforming photo-energy to electrical energy or a device for transforming electrical energy to photo-energy conversely. As examples, the organic photoelectric device includes an organic light emitting diode (OLED), a solar cell, a transistor, and the like. Particularly, an organic light emitting diode has recently drawn attention due to the increase in demand for flat panel displays.

US 2007/141390 A1 teaches a new class of bifunctional blue emitting materials for organic electroluminescent (EL) applications. These compounds contain two functionalities: an emission chromophore comprised of an anthryl group and an electron transport group.

EP 1 690 847 A1 teaches a π-conjugated compound having a cardo structure of formula (1):_ 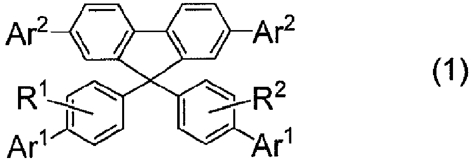 where R1 and R2 are hydrogen, alkyl, alkoxy, phenyl, naphthyl, phenoxy or halogen; Ar1 is a group of formula (2) or (3):_ 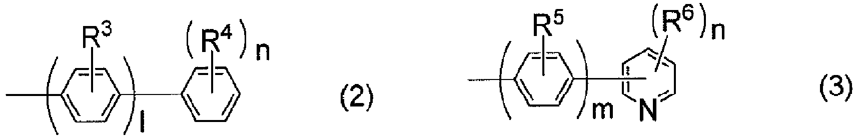 where R3 to R6 are hydrogen, alkyl, alkoxy, aryl, hetero-_aryl, aryloxy or a halogen, and 1 and m are 0-3, and n is 0-2;
and Ar2 is aryl or hetero aryl.

EP 1 400 578 A1 teaches arylamine derivatives that can be utilized as hole transport or hole injection materials of organic electroluminescence devices, electrophotographic reactors, etc., and synthetic intermediates thereof, and processes of producing those. The arylamine derivative is represented by the general formula (1): 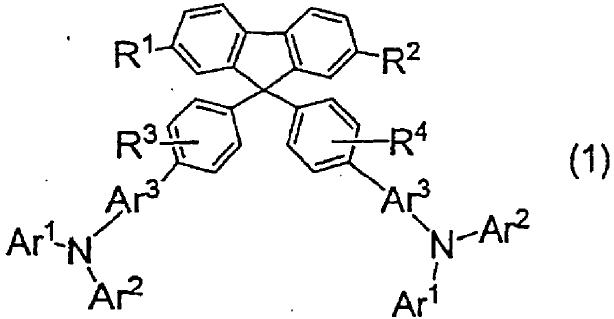 wherein R1 to R4 each independently represents a hydrogen atom, an alkyl group, an alkoxy group, an aryl group, an aryloxy group, a halogen atom, an amino group, etc.; Ar1 and Ar2 each independently represents a substituted or unsubstituted aryl group or hetero-aromatic group, and Ar1 and Ar2 may form a nitrogen-containing heterocyclic ring together with the nitrogen atom to which Ar1 and Ar2 bond; and Ar3 represents a substituted or unsubstituted arylene group.

When current is applied to an organic light emitting diode, holes are injected from an anode and electrons are injected from a cathode, then injected holes and electrons move to each hole transport layer (HTL) and electron transport layer (ETL) and recombined to a light emitting exciton in an emission layer. The light emitting excitons generate lights while shifting to a ground state. The light emission material may be classified as a fluorescent material including singlet excitons and a phosphorescent material including triplet excitons according to light emitting mechanism. The fluorescent and phosphorescent materials may be used for a light emitting source of an organic light emitting diode (D. F.O'Brien, Appl. Phys. Lett., 74 3, 442, 1999; M. A. Baldo, Appl. Phys. lett., 75 1, 4, 1999).

When electrons are transported from the ground state to the exited state, a singlet exciton undergoes non-light emitting transition to a triplet exciton through intersystem crossing and the triplet exciton is transited to the ground state to emit light. Herein, such a light emission refers to phosphorescent emission. When the triplet exciton is transited, it cannot directly transit to the ground state. Therefore, it is transited to the ground state after the electron spin is flipped

Therefore, a half-life (light emitting time, lifetime) of phosphorescent emission is longer than that of fluorescent emission.

When holes and electrons are recombined to produce a light emitting exciton, three times triplet light emitting excitons are produced compared to the amount of the singlet light emitting excitons. A fluorescent material has 25% of the singlet-exited state and a limit in luminous efficiency. On the other hand, a phosphorescent material can utilize 75% of the triplet exited state and 25% of the singlet exited state, so it can theoretically reach 100% of the internal quantum efficiency. Accordingly, the phosphorescent light emitting material has advantages of accomplishing around four times more luminous efficiency than the fluorescent light emitting material.

Meanwhile, a dopant along with the host material may be included in an emission layer to increase efficiency and stability of organic light emitting diode. For the host material, 4-N,N-dicarbazolebiphenyl (CBP) has been mainly used. However, CBP has high structural symmetry and may be easily crystallized. Due to low thermal stability, short-circuit or a pixel defect may occur during heat resistance test of a device. Furthermore, since host materials such as CBP has faster hole transport speed than electron transport speed, an exciton may not be effectively formed in an emission layer, decreasing luminous efficiency of a device.

In addition, a low molecular host material is in general using a vacuum-deposition, which may cost more than a wet process. In addition, most of low molecular host materials have low solubility against an organic solvent, they may not be applied in a wet process and thus, form an organic thin layer having excellent film characteristics.

Accordingly, in order to realize an organic photoelectric device with excellent efficiency and life-span, there are needs for development of a phosphorescent host material and a charge transport material having excellent electrical and thermal stability and bipolar characteristics well-transporting both holes and electrons or a host material mixed with a material being capable of transporting holes and electrons well.

### [DISCLOSURE]

### [Technical Problem]

A compound for an organic photoelectric device having excellent thermal stability, and being capable of transporting holes and electrons well is provided.

### [Technical Solution]

An organic photoelectric device having excellent efficiency and a driving voltage by including the compound for an organic photoelectric device is also provided.

A display device including the organic photoelectric device is also provided.

According to one aspect of the present invention, a compound for an organic photoelectric device represented by the following Chemical Formula 1 is provided. 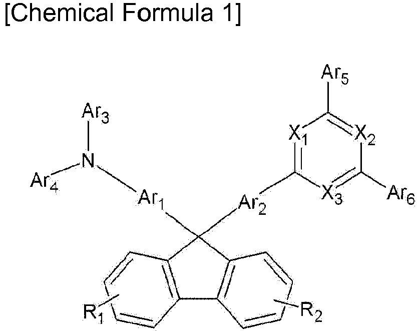 In Chemical Formula 1,
X₁ to X₃ are the same or different, and are each independently N or CR, provided that at least one of X₁ to X₃ is N, and R is hydrogen or a C1 to C10 lower alkyl group,
Ar₁ and Ar₂ are the same or different, and are each independently substituted or unsubstituted C6 to C18 arylene group, wherein the substituent of Ar₁ and Ar₂ is selected the group consisting of a C1 to C30 alkyl group, a C1 to C10 alkylsilyl group, a C3 to C30 cycloalkyl group, a C6 to C30 aryl group, a C1 to C10 alkoxy group, a fluoro group, a C1 to C10 trifluoroalkyl group, and a cyano,and
Ar₃ and Ar₄ are the same or different, and are each independently substituted or unsubstituted C1 to C30 alkyl group, a substituted or unsubstituted C1 to C30 alkylene group, a substituted or unsubstituted C6 to C30 aryl group, a substituted or unsubstituted C6 to C30 arylene group, a substituted or unsubstituted C2 to C30 heteroaryl group, a substituted or unsubstituted C2 to C30 heteroarylene group, or a combination thereof, or Ar₃ and Ar₄ are fused to each other to provide a fused ring, wherein the substituent of Ar₃ and Ar₄ is selected the group consisting of a C1 to C30 alkyl group, a C1 to C10 alkylsilyl group, a C3 to C30 cycloalkyl group, a C6 to C30 aryl group, a C1 to C10 alkoxy group, a fluoro group, a C1 to C10 trifluoroalkyl group, and a cyano,
Ar₅ and Ar₆ are the same or different, and are each independently substituted or unsubstituted C1 to C30 alkyl group, a substituted or unsubstituted C6 to C30 aryl group, a substituted or unsubstituted C2 to C30 heteroaryl group, or a combination thereof, wherein the substituent of Ar₅ and Ar₆ is selected the group consisting of a C1 to C30 alkyl group, a C1 to C10 alkylsilyl group, a C3 to C30 cycloalkyl group, a C6 to C30 aryl group, a C1 to C10 alkoxy group, a fluoro group, a C1 to C10 trifluoroalkyl group, and a cyano, and
R₁ and R₂ are the same or different, and are each independently hydrogen or a C1 to C10 lower alkyl group.

All of the X₁ to X₃ of the above Chemical Formula 1 are N.

In Chemical Formula 1 the arylene group of Ar₁ and Ar₂ may be a phenylene group, a naphthylene group, an anthracenylene group, or a combination thereof, and the aryl group of Ar₃ to Ar₆ may be a phenyl group, a naphthyl group, an anthracene group, a phenanthrene group, a tetracene group, a pyrene group, a fluorene group, or a combination thereof, and particularly, the aryl group of Ar₃ to Ar₆ may be a substituent represented by the following Chemical Formulas 2a to 2c, or a combination thereof.

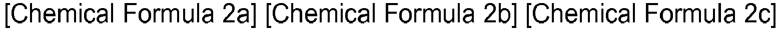

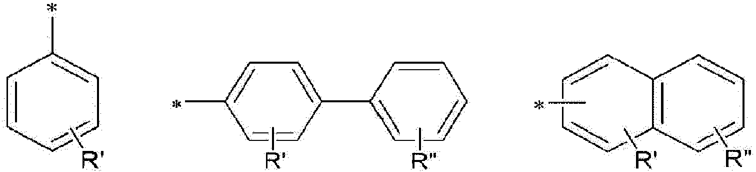

In Chemical Formulas 2a to 2c,
R' and R" are the same or different, and are each independently hydrogen, a C1 to C10 lower alkyl group, a C6 to C18 aryl group, or a combination thereof.

The heteroaryl group of Ar₅ and Ar₆ in Chemical Formula 1 may be furan, pyrrole, imidazole, thiazole, oxazole, oxadiazole, thiadiazole, triazole, triazine, pyridine, pyrimidine, pyridazine, pyrazine, quinoline, isoquinoline, or a combination thereof.

The compound for an organic photoelectric device may be represented by the following Chemical Formulas 3a or 3b.

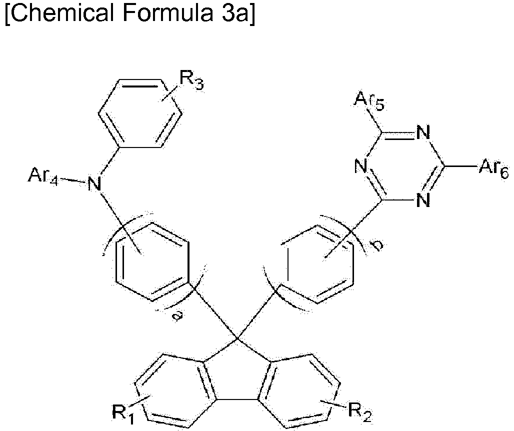

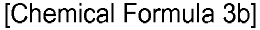

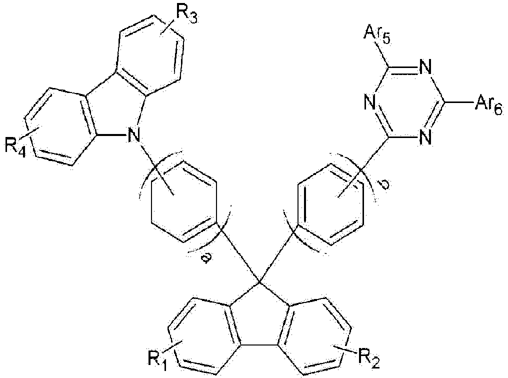

In Chemical Formulas 3a and 3b,
Ar₄ to Ar₆ are the same or different, and are each independently substituted or unsubstituted C1 to C30 alkyl group, a substituted or unsubstituted C6 to C30 aryl group, a substituted or unsubstituted C2 to C30 heteroaryl group, or a combination thereof,
R₁ and R₂ are the same or different, and are each independently hydrogen or a C1 to C10 lower alkyl group,
R₃ and R₄ are the same or different, and are each independently hydrogen, a C1 to C10 lower alkyl group, a C6 to C18 aryl group, or a combination thereof, and
a and b are the same or different, and are each independently 1 or 2.

The compound for an organic photoelectric device are represented by the following Chemical Formulas 4 to 35 and ad-1 to ad-4.

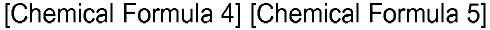

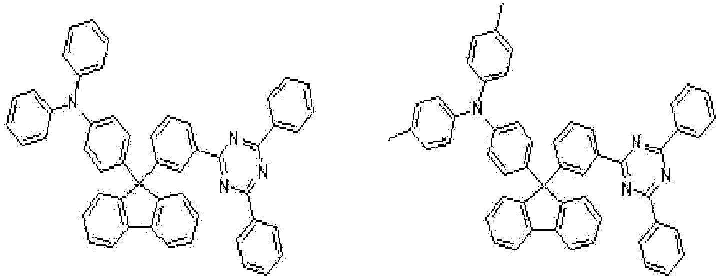

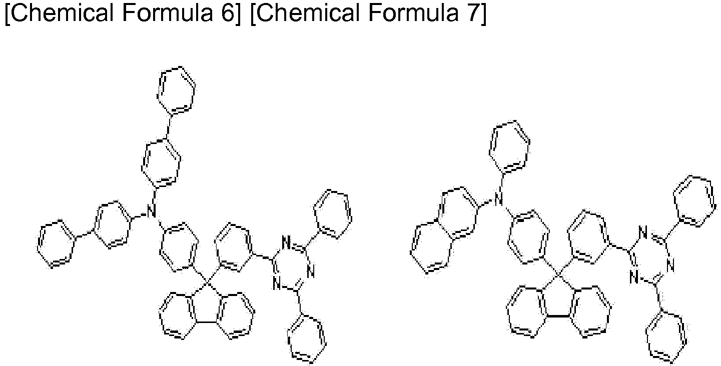

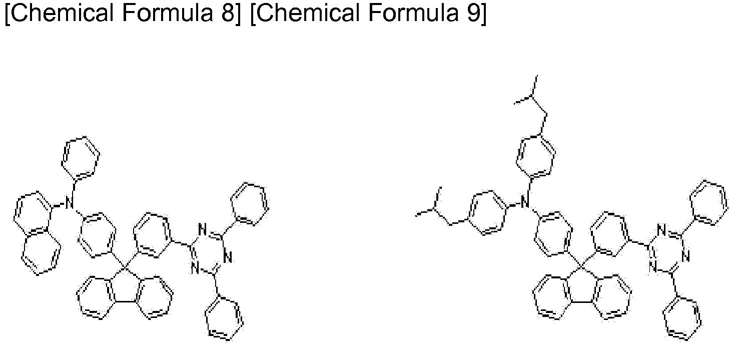

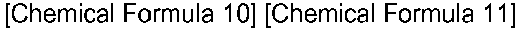

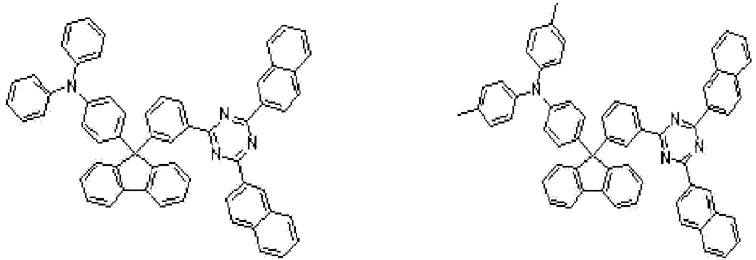

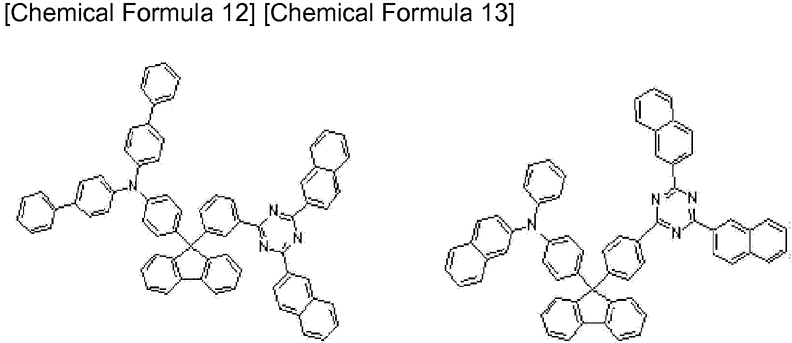

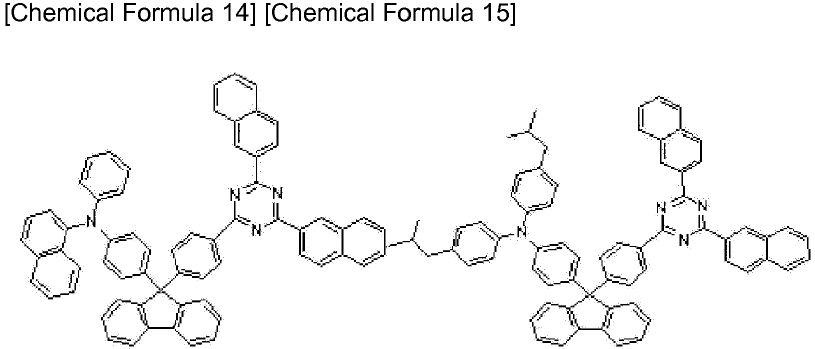

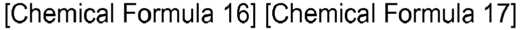

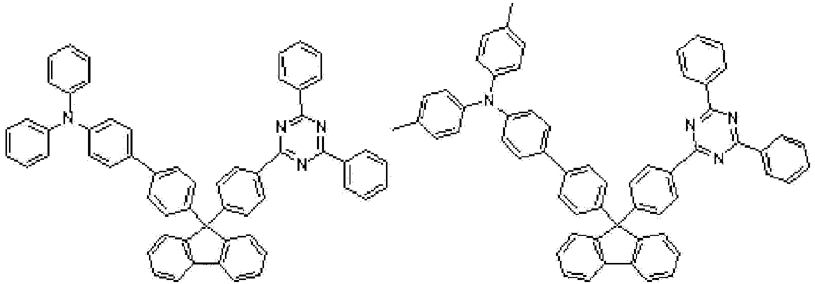

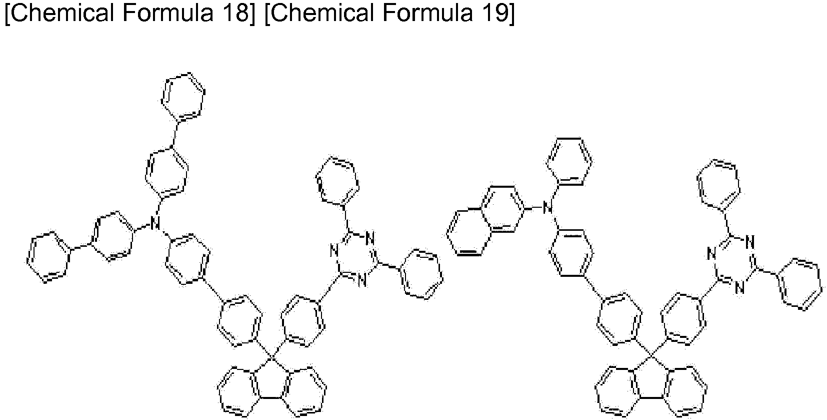

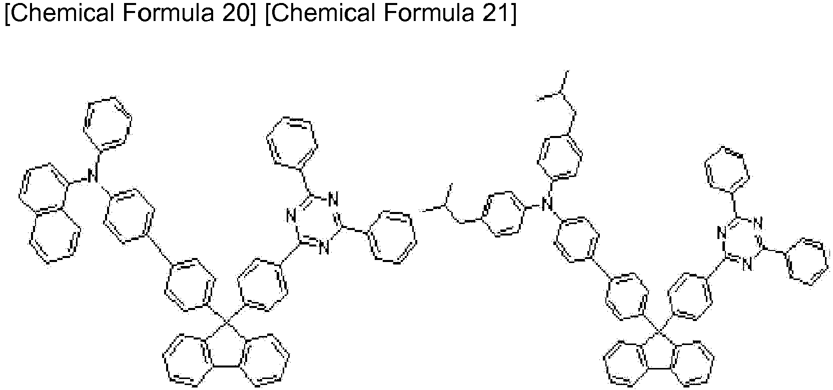

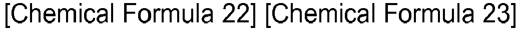

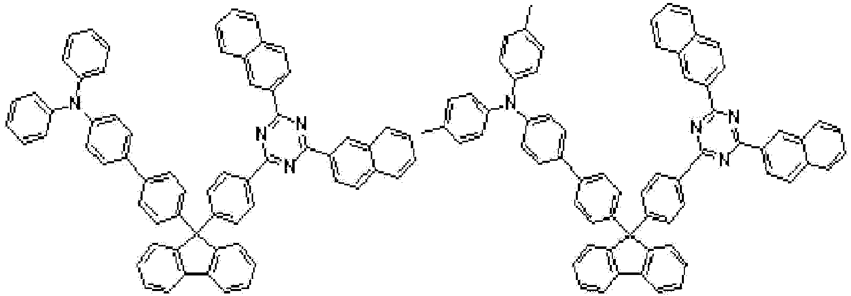

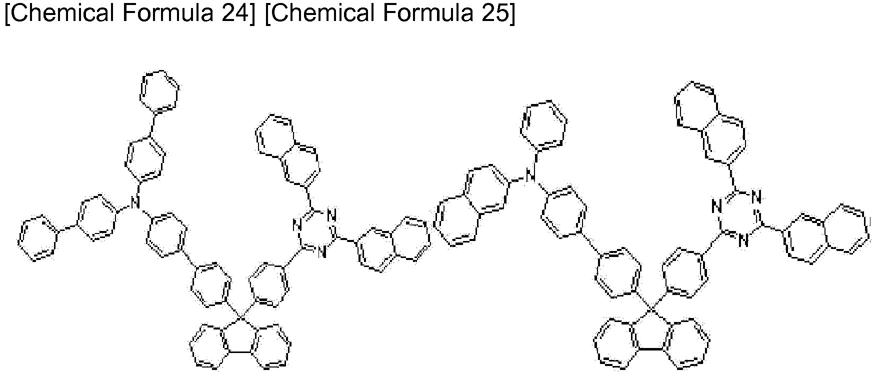

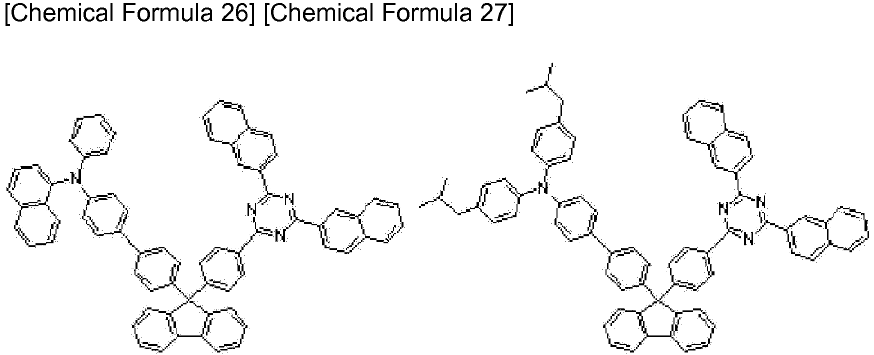

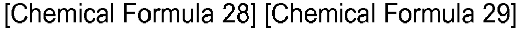

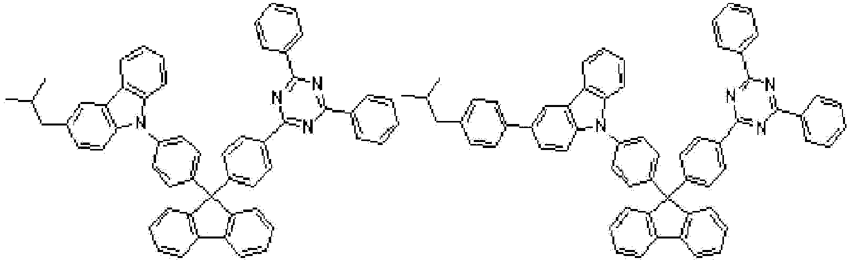

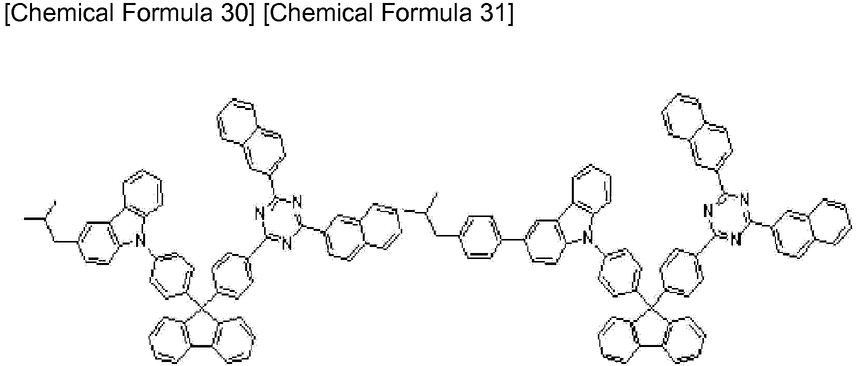

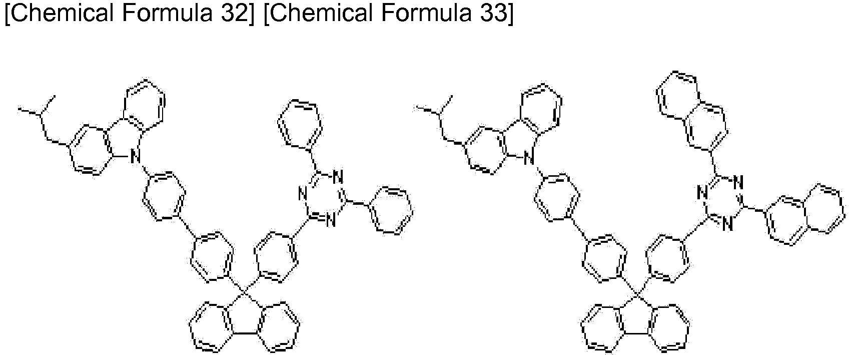

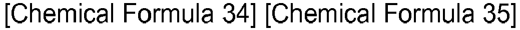

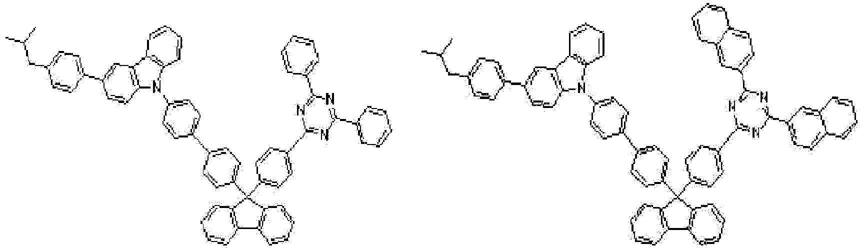

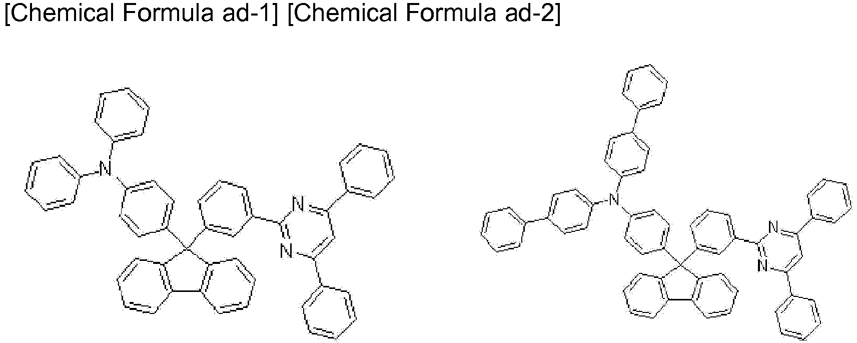

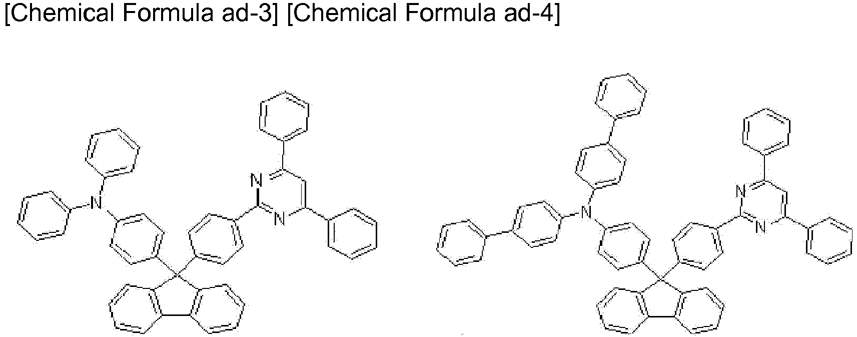

The compound for an organic photoelectric device may be used as a charge transport material or a host material, and the compound for an organic photoelectric device may have a glass transition temperature (Tg) of 110 ° C or more and a thermal decomposition temperature (Td) of 400 ° C or more.

According to another embodiment of the present invention, provided is a organic photoelectric device that includes an anode, a cathode and at least one or more organic thin layer between the anode and the cathode, wherein the organic thin layer includes the compound for an organic photoelectric device.

The organic thin layer may be an emission layer, a hole transport layer (HTL), a hole injection layer (HIL), a hole blocking layer, an electron transport layer (ETL), an electron injection layer (EIL), an electron blocking layer, or a combination thereof, the organic thin layer may further include a dopant, and the dopant may be a red, green, blue or white phosphorescent dopant.

According to yet another embodiment of the present invention, a display device including the organic photoelectric device is provided.

Hereinafter, further embodiments of the present invention will be described in detail.

### [Advantageous Effects]

The compound for an organic photoelectric device according to one aspect has excellent thermal stability, and particularly, may be applied to an organic thin layer of an organic photoelectric device and thus can provide an organic photoelectric device and a display device having high luminous efficiency at a low voltage and improved life-span.

### [Description of the Drawings]

FIGS. 1 to 5 are cross-sectional views showing organic photoelectric devices including compounds according to various embodiments of the present invention.
FIG. 6 is a graph showing DSC (differential scanning calorimetry) analysis results of the compounds for an organic photoelectric device according to Examples 1 to 3.

### <Description of Reference Numerals Indicating Primary Elements in the Drawings>

100: organic photoelectric device 110: cathode
120: anode 105: organic thin layer
130: emission layer 140: whole transport layer (HTL)
150: electron transport layer (ETL) 160: electron injection layer (EIL)
170: hole injection layer (HIL) 230: emission layer + electron transport layer (ETL)

### [Mode for Invention]

Exemplary embodiments of the present invention will hereinafter be described in detail. However, these embodiments are only exemplary, and the present invention is not limited thereto but rather is defined by the scope of the appended claims.

In the present specification, the term "substituted", when a definition is not otherwise provided, refers to one substituted with a C1 to C30 alkyl group; a C1 to C10 alkylsilyl group; a C3 to C30 cycloalkyl group; a C6 to C30 aryl group; a C1 to C10 alkoxy group; a fluoro group, a C1 to C10 trifluoroalkyl group such as a trifluoromethyl group, and the like; or a cyano group.

In the present specification, the term "hetero", when a definition is not otherwise provided, refers to one including 1 to 3 of N, O, S, and P, and remaining carbons in a compound or a substitutent.

In the present specification, the term "combination thereof', when a definition is not otherwise provided, refers to at least two substituents bound to each other, or at least two substituents condensed to each other.

In the specification, the term "alkyl group" when a definition is not otherwise provided, refers to a "saturated alkyl group" that does not include any alkene group or alkyne group; or an "unsaturated alkyl group" that includes at least one alkene group or alkyne group. The term "alkene group" refers to a group in which at least two carbon atoms are bound with at least one carbon-carbon double bond, and the term "alkyne group" refers to a group in which at least two carbon atoms are bound with at least one carbon-carbon triple bond. The alkyl group may be branched, linear, or cyclic.

The alkyl group may be a C1 to C20 alkyl group, and more particularly a C1 to C6 lower alkyl group, a C7 to C10 middle alkyl group, or a C11 to C20 higher alkyl group.

For example, a C1 to C4 alkyl group includes 1 to 4 carbon in an alkyl chain and may be selected from the group consisting of methyl, ethyl, propyl, iso-propyl, n-butyl, iso-butyl, sec-butyl and t-butyl.

Examples of an alkyl group may be a methyl group, an ethyl group, a propyl group, an isopropyl group, a butyl group, an isobutyl group, a t-butyl group, a pentyl group, a hexyl group, an ethenyl group, a propenyl group, a butenyl group, a cyclopropyl group, a cyclobutyl group, a cyclopentyl group, a cyclohexyl group, and the like.

The term "aromatic group" refers to a cyclic functional group where all elements have conjugated p-orbital. Examples of the aromatic group include an aryl group and a heteroaryl group.

The term "aryl" group refers to a monocyclic or fused cyclic substituent where adjacent pairs of carbon atoms are shared.

The term "heteroaryl group" may refer to an aryl group where 1 to 3 heteroatoms selected from N, O, S, and P, and remaining carbon. When the heteroaryl group is a fused ring, each ring may include 1 to 3 heteroatoms.

The term "spiro structure" may refer to a cyclic structure having a common carbon contact point. The term spiro structure may refer to a compound or substituent having a spiro structure.

According to one embodiment of the present invention, a compound of Chemical Formulas 1 to 35 and ad-1 to ad-4 for an organic photoelectric device represented by the following Chemical Formula 1 is provided.

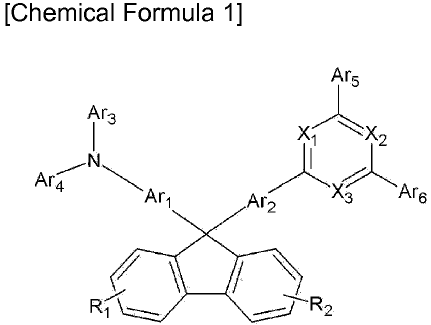

In Chemical Formula 1,
X₁ to X₃ are the same or different, and are each independently N or CR, provided that at least one of X₁ to X₃ is N, and R is hydrogen or a C1 to C10 lower alkyl group,
Ar₁ and Ar₂ are the same or different, and are each independently substituted or unsubstituted C6 to C18 arylene group,
Ar₃ and Ar₄ are the same or different, and are each independently substituted or unsubstituted C1 to C30 alkyl group, a substituted or unsubstituted C1 to C30 alkylene group, a substituted or unsubstituted C6 to C30 aryl group, a substituted or unsubstituted C6 to C30 arylene group, a substituted or unsubstituted C2 to C30 heteroaryl group, a substituted or unsubstituted C2 to C30 heteroarylene group, or a combination thereof, the Ar₃ and Ar₄ are fused to each other to provide a fused ring,
Ar₅ and Ar₆ are the same or different, and are each independently substituted or unsubstituted C1 to C30 alkyl group, a substituted or unsubstituted C6 to C30 aryl group, a substituted or unsubstituted C2 to C30 heteroaryl group, or a combination thereof, and
R₁ and R₂ are the same or different, and are each independently hydrogen or a C1 to C10 lower alkyl group.

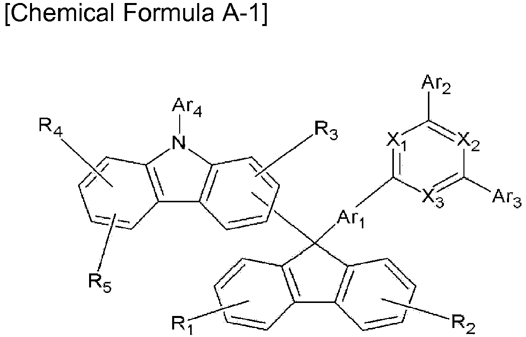

The compound represented by the above Chemical Formula 1 includes a substituent represented by the following Chemical Formula 1 a or a carbazolyl group; and a substituent represented by the following Chemical Formula 1b and thus, may have bipolar characteristic of well transporting both holes and electrons.

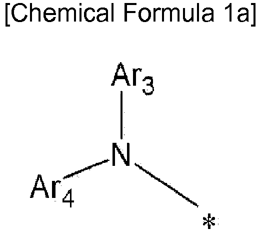

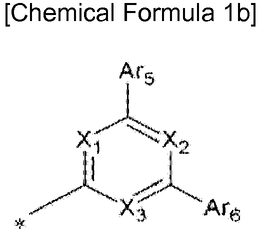

In Chemical Formulas 1 a and 1 b, X₁ to X₃ and Ar₃ to Ar₆ are the same as defined in Chemical Formula 1.

Particularly, the substituent represented by the above Chemical Formula 1 a or the carbazolyl group plays a role of a group injecting and transporting a hole, while the substituent represented by the above Chemical Formula 1 b plays a role of a group injecting and transporting an electron. The group injecting and transporting a hole has conductivity characteristic along a HOMO level and thus, delivers and injects a hole produced from an anode into an emission layer. In addition, the group injecting and transporting an electron has conductivity characteristic along a LUMO level and thus, delivers and injects a hole produced from a cathode into an emission layer.

Accordingly, a compound for an organic photoelectric device according to one embodiment of the present invention may have bipolar characteristic. In other words, the compound for an organic photoelectric device is amphiphilic and thus, excellent interface characteristic and charge transporting capability in an emission layer of the organic photoelectric device, in which holes are combined with electrons.

Furthermore, a substituent represented by the above Chemical Formula 1a or a carbazolyl group; and another substituent represented by Chemical Formula 1 b are not conjugated with each other and thus, have no influence on each other's inherent hole-injecting and transporting and electron-injecting and transporting characteristics, even though these substuents are combined in one compound. In general, a compound with a long conjugated structure may have an energy level change, which may change hole and electron injection/transportation characteristic. However, a compound for an organic photoelectric device according to one embodiment of the present invention includes a substituent represented by the above Chemical Formula 1a or a carbazolyl group; and another substituent represented by Chemical Formula 1 b which are not conjugated and thus, has excellent bipolar characteristic.

In addition, the compound simultaneously including the substituent represented by the above Chemical Formula 1 a or the carbazolyl group, above Chemical Formula 1 a; and the substituent represented by Chemical Formula 1 b and Chemical Formula 1 b has an asymmetrical structure and may be prevented from easy crystallization. In other words, the compound for an organic photoelectric device according to one embodiment of the present invention may be usefully applied as a host material, hole transport material, or electron transport material with excellent thermal stability.

In Chemical Formula 1, X₁ to X₃ are the same or different, and are each independently N or CR, provided that at least one of X₁ to X₃ is N, and R is hydrogen or a C1 to C10 lower alkyl group. Herein, when all of the X₁ to X₃ are N, the compound may better transport electrons.

The arylene group of Ar₁ and Ar₂ of the Chemical Formula 1 may be a phenylene group, a naphthylene group, an anthracenylene group, or a combination thereof. However, the arylene group is not limited thereto.

In addition, when Ar₃ to Ar₆ in the above Chemical Formula 1 are independently an aryl group, the compound includes a C1 to C10 lower alkyl group, a C6 to C18 aryl group, or a combination thereof as a substituent and thus, may maintain dissolving properties in a solvent and stable bipolar characteristic.

Particularly, the aryl group of Ar₃ to Ar₆ of the above Chemical Formula 1 may be a phenyl group, a naphthyl group, an anthracene group, a phenanthrene group, a tetracene group, a pyrene group, a fluorene group, or a combination thereof. However, the aryl group is not limited thereto.

Additionally, the aryl group of Ar₃ to Ar₆ of the above Chemical Formula 1 may be a substituent represented by the following Chemical Formulas 2a to 2c, or a combination thereof.

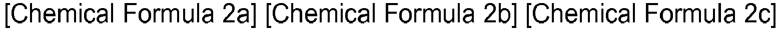

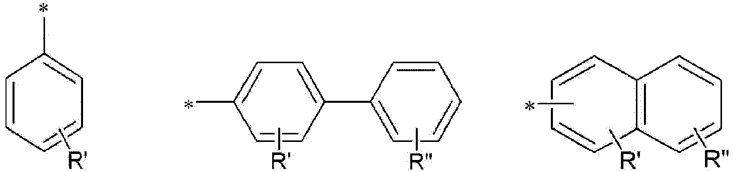

In Chemical Formulas 2a to 2c,
R' and R" are the same or different, and are each independently hydrogen, a C1 to C10 lower alkyl group, a C6 to C18 aryl group, or a combination thereof.

In addition, a substituent R' in the above Chemical Formula 2a may be boound at a para position.

Furthermore, a heteroaryl group of Ar₅ and Ar₆ in the above Chemical Formula 1 include 1 to 3 hetero atoms consisting of N, O, S, P, or a combination thereof in each cyclic group and a remaining carbon. The hetero aryl group has no particular limit but necessarily includes N and thus, helps a substituent represented by the above Chemical Formula 1 b transport electrons better. Examples of the heteroaryl group of Ar₅ and Ar₆ in Chemical Formula 1 may be furan, pyrrole, imidazole, thiazole, oxazole, oxadiazole, thiadiazole, triazole, triazine, pyridine, pyrimidine, pyridazine, pyrazine, quinoline, isoquinoline, or a combination thereof. However, the heteroaryl group is not limited thereto.

The compound for an organic photoelectric device of Chemical Formulas 1 to 35 and ad-1 to ad-4 may be represented by the following Chemical Formulas 3a or 3b.

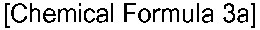

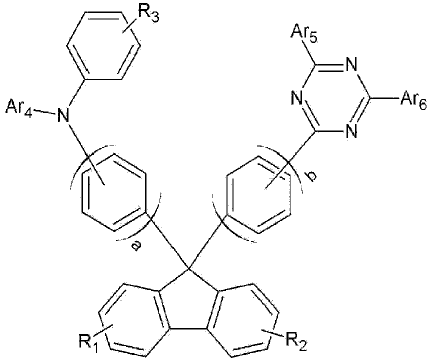

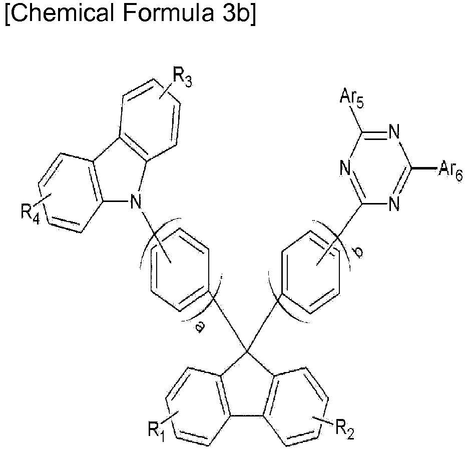

In Chemical Formulas 3a and 3b,
Ar₄ to Ar₆ are the same or different, and are each independently substituted or unsubstituted C1 to C30 alkyl group, a substituted or unsubstituted C6 to C30 aryl group, a substituted or unsubstituted C2 to C30 heteroaryl group, or a combination thereof,
R₁ and R₂ are the same or different, and are each independently hydrogen or a C1 to C10 lower alkyl group,
R₃ and R₄ are the same or different, and are each independently hydrogen, a C1 to C10 lower alkyl group, a C6 to C18 aryl group, or a combination thereof, and
a and b are the same or different, and are each independently 1 or 2.

The compound for an organic photoelectric device according to one embodiment may be one selected from the group consisting of the above Chemical Formulas 4 to 35 and Chemical Formulas ad-1 to ad-4. However, the present invention is not limited to the compounds.

The compound for an organic photoelectric device according to one embodiment may be one selected from the group consisting of the above Chemical Formulas ad-5 to ad-24. However, the present invention is not limited to the compounds.

Therefore, a compound for an organic photoelectric device according to one embodiment of the present invention is used for an organic thin layer and generates phosphorescence in a wide wavelength region and thus, may improve efficiency characteristic of the organic photoelectric device and lower its driving voltage. In addition, the compound may improve life-span characteristic of the organic photoelectric device.

The compound for an organic photoelectric device may be a charge transporting material having excellent hole and electron transportation capability. In addition, the compound for an organic photoelectric device may be used singularly but as a host material with a dopant together.

The dopant is a compound having a high emission property, by itself. However, it is usually added to a host in a minor amount, so it is also called a guest. The dopant may be a light-emitting material while being doped on a host material. Generally, a material such as a metal complex being capable of light-emitting by multiplet excitation such as triplet excitation or more is used for a dopant. Such a dopant may be a generally-used red (R), green (G), blue (B), or white (W) fluorescent or phosphorescent dopant, and particularly, red, green and blue, or white phosphorescent dopant is preferable. A material that has high luminous efficiency, is not agglomerated, and is distributed in a host material uniformly may be used.

The phosphorescent dopant may be an organic metal compound including an element that is Ir, Pt, Os, Ti, Zr, Hf, Eu, Tb, Tm, Fe, Co, Ni, Ru, Rh, Pd, or a combination thereof. More particularly, a red phosphorescent dopant may include platinum-octaethylporphyrin complex (PtOEP), Ir(Piq)₃, RD61 (UDC), and the like, a green phosphorescent dopant may include Ir(PPy)₂(acac), Ir(PPy)₃, Ir(mppy)₃, GD48 (UDC), and the like, and a blue phosphorescent dopant may include (4,6-F₂PPy)₂Irpic (Appl. Phys. Lett.,79, 2082-2084, 2001), and the like. The "Piq" denotes 1-phenylisoquinoline, "acac" denotes acetylacetonate, PPy denotes 2-phenylpyridine, pic denotes picolinate, and mppy denotes 2- (4-methylphenyl)pyridine.

The compound for an organic photoelectric device according to one embodiment of the present invention has a glass transition temperature (Tg) of 110 ° C or more, and a thermal decomposition temperature (Td) of 400 ° C or more, and more particularly a glass transition temperature of 110 to 200 ° C, and a thermal decomposition temperature of 400 to 600 ° C. Thereby, the compound for an organic photoelectric device according to one embodiment of the present invention has excellent thermal stability and may be used as a host material or a charge transport material.

According to another embodiment of the present invention, an organic photoelectric device including the compound for an organic photoelectric device is provided. The organic photoelectric device may be an organic light emitting diode, an organic solar cell, an organic transistor, an organic photo conductor drum, an organic memory device, and the like. In case of an organic solar cell, the compound for an organic photoelectric device according to one embodiment may be applied to an electrode or an electrode buffer layer of an organic solar cell to improve quantum efficiency, or may be applied to an electrode material of a gate, source-drain electrodes, and the like of an organic transistor.

Hereinafter, an organic photoelectric device is illustrated in more detail.

According to another embodiment of the present invention, an organic photoelectric device includes an anode, a cathode and at least one or more organic thin layer between the anode and the cathode, wherein the organic thin layer includes the compound for an organic photoelectric device according to one embodiment.

The organic thin layer including the compound for an organic photoelectric device may be an emission layer, a hole transport layer (HTL), a hole injection layer (HIL), a hole blocking layer, an electron transport layer (ETL), an electron injection layer (EIL), an electron blocking layer, or a combination thereof. At least one of these layers includes the compound for an organic photoelectric device according to one embodiment of the present invention. More particularly, an emission layer, a hole transport layer (HTL), a hole injection layer (HIL), an electron transport layer (ETL), an electron injection layer (EIL), or a combination thereof may include the compound for an organic photoelectric device according to one embodiment of the present invention.

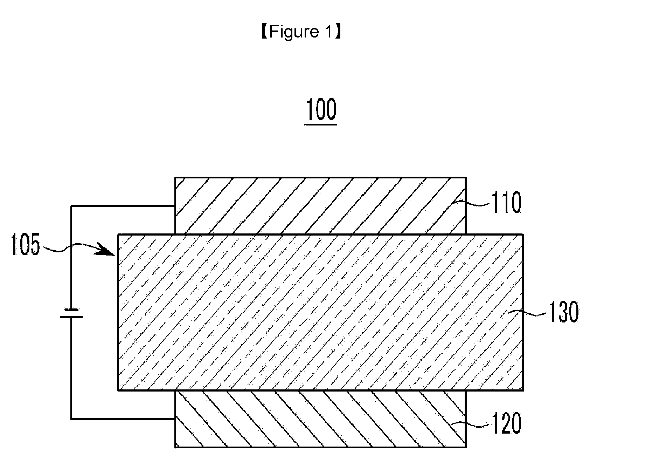

FIGS. 1 to 5 are cross-sectional views showing organic photoelectric devices including the compound for an organic photoelectric device.

Referring to FIGS. 1 to 5, the organic photoelectric devices 100, 200, 300, 400, and 500 according to one embodiment includes at least one organic thin layer 105 interposed between an anode 120 and a cathode 110.

A substrate of an organic photoelectric device is not particularly limited in the related arts, but a glass substrate or a transparent plastic substrate having excellent transparency, surface smoothness, handling ease, and water repellency.

The anode 120 includes an anode material laving a large work function to help hole injection into an organic thin layer. The anode material includes a metal such as nickel, platinum, vanadium, chromium, copper, zinc, gold, and the like or an alloy of the foregoing metals; metal oxide such as zinc oxide, indium oxide, indium tin oxide (ITO), indium zinc oxide (IZO), and the like; a combined metal and oxide such as ZnO/Al, SnO₂/Sb, and the like; a conductive polymer such as poly (3-methylthiophene), poly[3,4-(ehtylene-1,2-dioxy)thiophene]: (PEDOT or PEDT), polypyrrole, polyaniline, and the like. But the anode material is not limited thereto. Particularly, the anode material may be a transparent electrode including ITO.

The cathode 110 includes a cathode material having a small work function to help electron injection into an organic thin layer. The cathode material includes a metal such as magnesium, calcium, sodium, potassium, titanium, indium, yttrium, lithium, gadolinium, aluminum, silver, tin, lead, cesium, barium, and the like or alloys thereof; a multi-layered material such as LiF/Al, LiO₂/Al, LiF/Ca, LiF/Al, BaF₂/Ca, and the like. But the cathode material is not limited to the above materials. Particularly, the cathode material may be a metal electrode such as aluminum.

Referring to FIG. 1, the organic photoelectric device 100 includes an organic thin layer 105 including only an emission layer 130.

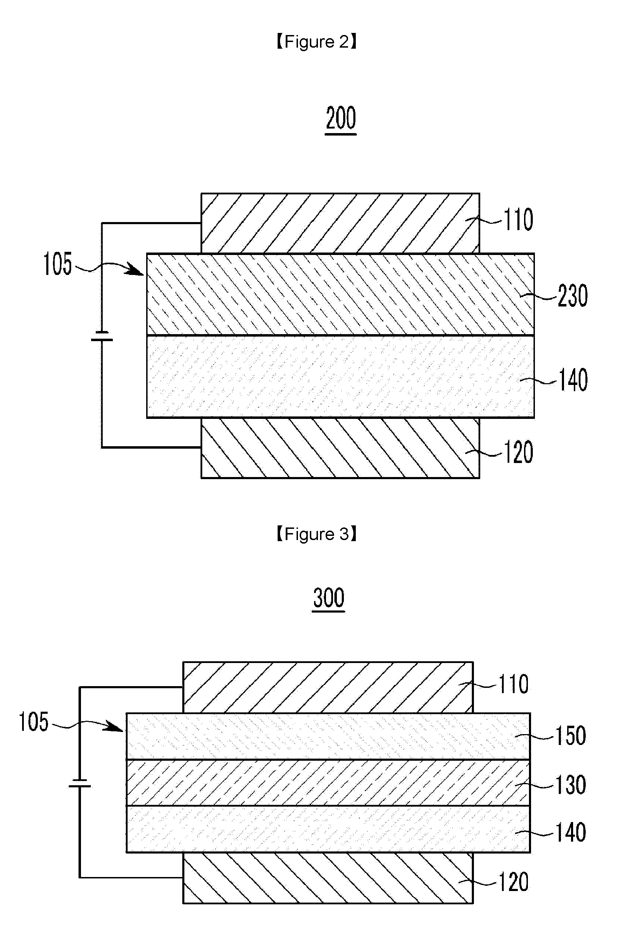

Referring to FIG. 2, a double-layered organic photoelectric device 200 includes an organic thin layer 105 including an emission layer 230 including an electron transport layer (ETL), and a hole transport layer (HTL) 140. The emission layer 130 also functions as an electron transport layer (ETL), and the hole transport layer (HTL) 140 layer has an excellent binding property with a transparent electrode such as ITO or an excellent hole transporting property.

The hole transport layer (HTL) 140 may include generally-used hole transport material without limitation, for example poly (3,4-ethylenedioxy-thiophene) (PEDOT) doped with poly(styrenesulfonate) (PSS) (PEDOT:PSS), N,N'-bis (3-methylphenyl)-N,N-diphenyl-[1,1'-biphenyl]-4,4'-diamine (TPD), N,N'-di (1-naphthyl)-N,N'-diphenylbenzidine (NPB) and the like, along with the compound for an organic photoelectric device according to one embodiment of the present invention.

Referring to FIG. 3, a three-layered organic photoelectric device 300 includes an organic thin layer 105 including an electron transport layer (ETL) 150, an emission layer 130, and a hole transport layer (HTL) 140. The emission layer 130 is independently installed, and layers having an excellent electron transporting property or an excellent hole transporting property are separately stacked.

The electron transport layer (ETL) 150 may include generally-used electron transport material without limitation, for example, aluminum tris(8-hydroxyquinoline) (Alq₃); a 1,3,4-oxadiazole derivative such as 2-(4-biphenyl-5-phenyl-1,3,4-oxadiazole (PBD); a quinoxalin derivative such as 1,3,4-tris[(3-phenyl-6-trifluoromethyl) quinoxalin-2-yl]benzene (TPQ); and a triazole derivative, along with the compound for an organic photoelectric device according to one embodiment of the present invention.

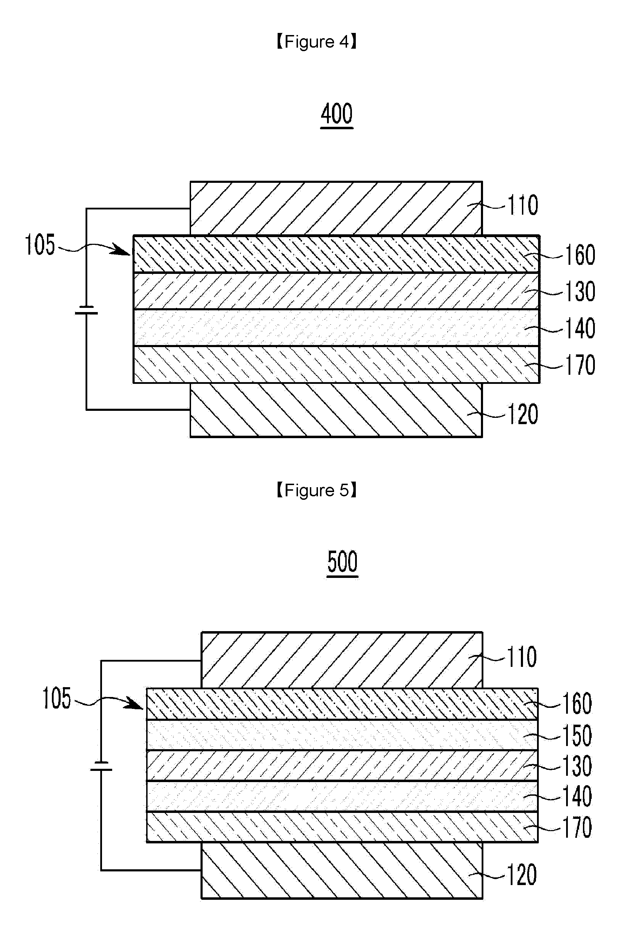

FIG. 4 shows a four-layered organic photoelectric device 400 that includes an organic thin layer 105 including an electron injection layer (EIL) 160, an emission layer 130, a hole transport layer (HTL) 140, and a hole injection layer (HIL) 170 for binding with the cathode of ITO.

FIG. 5 shows a five layered organic photoelectric device 500 that includes an organic thin layer 105 including an electron transport layer (ETL) 150, an emission layer 130, a hole transport layer (HTL) 140, and a hole injection layer (HIL) 170, and further includes an electron injection layer (EIL) 160 to achieve a low voltage.

The emission layers 130 and 230 may have a thickness of 5 to 1000 nm, the hole transport layer (HTL) 140 and electron transport layer (ETL) 150 may have a thickness of 10 to 10,000 Å, respectively. However, the thicknesses are not limited to the above range.

In FIGS. 1 to 5, the organic thin layer 105 selected from the electron transport layer (ETL) 150, electron injection layer (EIL) 160, emission layer 130 and 230, hole transport layer (HTL) 140, hole injection layer (HIL) 170 and a combination thereof includes the compound for an organic photoelectric device according to one embodiment. The material for the organic photoelectric device may be used for an electron transport layer (ETL) 150 including the electron transport layer (ETL) 150 or electron injection layer (EIL) 160. When it is used for the electron transport layer (ETL), it is possible to provide an organic photoelectric device having a simpler structure because it does not require an additional hole blocking layer.

Furthermore, when the compound for an organic photoelectric device is included in the emission layer 130 and 230, the material for the organic photoelectric device may be included as a phosphorescent host, and the emission layer 130 and 230 may further include a dopant. The dopant may be a red, green and blue, or white phosphorescent dopant.

The organic photoelectric device may be fabricated by: forming an anode on a substrate, forming an organic thin layer in accordance with a dry coating method such as evaporation, sputtering, plasma plating, and ion plating, or a wet coating method such as spin coating, dipping, and flow coating; and providing a cathode thereon.

Another embodiment of the present invention provides a display device including the organic photoelectric device.

Hereinafter, the embodiments are illustrated in more detail with reference to examples. However, the following are exemplary embodiments and are not limiting.

### Synthesis of Compound for Organic photoelectric device

### Example 1: Synthesis of Compound represented by Chemical Formula 4

A compound represented by the above Chemical Formula 4 presented as a specific example of a compound for an organic photoelectric device according to the present invention was synthesized in a method of Reaction Scheme 1.

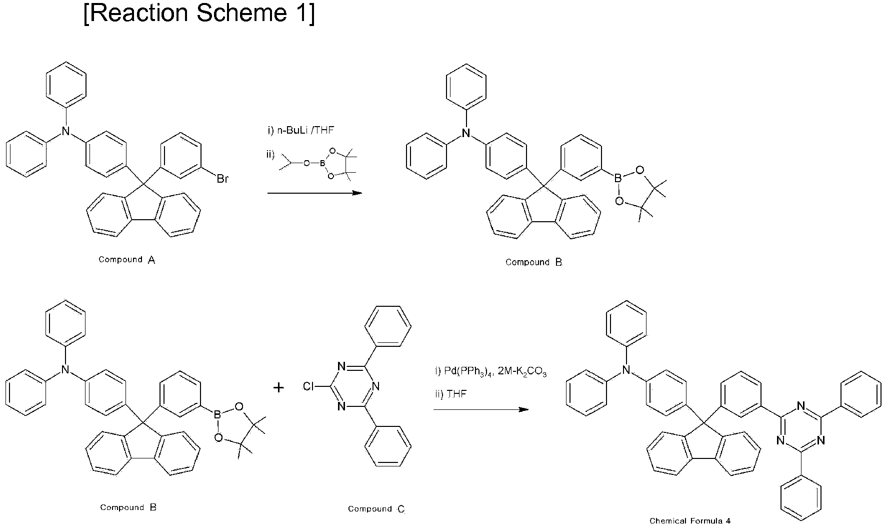

### First Step: Synthesis of Compound B

5 g (8.86 mmol) of the compound A is mixed with 150 mL of tetrahydrofuran in a 250 mL round-bottomed flask with an agitator under a nitrogen atmosphere and cooled down by using dry ice and acetone. Next, 6.64 mL (10.6 mmol) of 1.6 M n-butyllithium (n-BuLi) was slowly injected to the reactor to remove the dry ice, and then, the resulting product was agitated for 30 minutes. Then, the reactor was cooled down, and 2.47 g (13.3 mmol) of 2-isopropoxy-4,4,5,5-tetramethyl-1,3,2-dioxaborane was slowly injected thereto. The reactor was agitated at a room temperature for 8 hours.

The reactant was poured into water to complete the reaction. The reactant was extracted with ethylacetate and treated with anhydrousmagnesium sulfate to remove moisture and a solvent therein. The residue was purified through a silica gel chromatography, obtaining 4.11 g of an intermediate product (yield: 76 %) represented by a compound B.

### Second Step: Synthesis of Chemical Formula 4

4 g (6.5 mmol) of an intermediate product represented by the compound B and 2.1 g (7.85 mmol) of a compound C were mixed with 100 mL of tetrahydrofuran in a 250 mL round flask. Next, 80 mL of a 2M-potassium carbonate aqueous solution and 0 0.15 g (0.13 mmol) of tetrakistriphenylphosphine palladium (0) were added to the mixture. The resulting mixture was heated and refluxed under a nitrogen gas flow for 12 hours.

The liquid reactant was separated into two layers. The organic layer was cleaned with a sodium chloride saturated aqueous solution and dried with anhydrous sodium sulfate. Then, the organic solvent therein was distillated and removed under a reduced pressure. The residue was purified through a silica gel chromatography, obtaining 3.38 g of a compound represented by Chemical Formula 4 (yield: 72%).

The compound represented by Chemical Formula 4 was performed regarding atomic analysis. The result is provided as follows.
calcd. C₅₂H₃₆N₄: C, 87.12; H, 5.06; N, 7.82; found: C, 87.02; H, 5.11; N, 7.32.

### Example 2: Synthesis of Compound represented by Chemical Formula 23

A compound represented by the above Chemical Formula 23 as a specific example of a compound for an organic photoelectric device according to the present invention was synthesized according to the following Reaction Scheme 2.

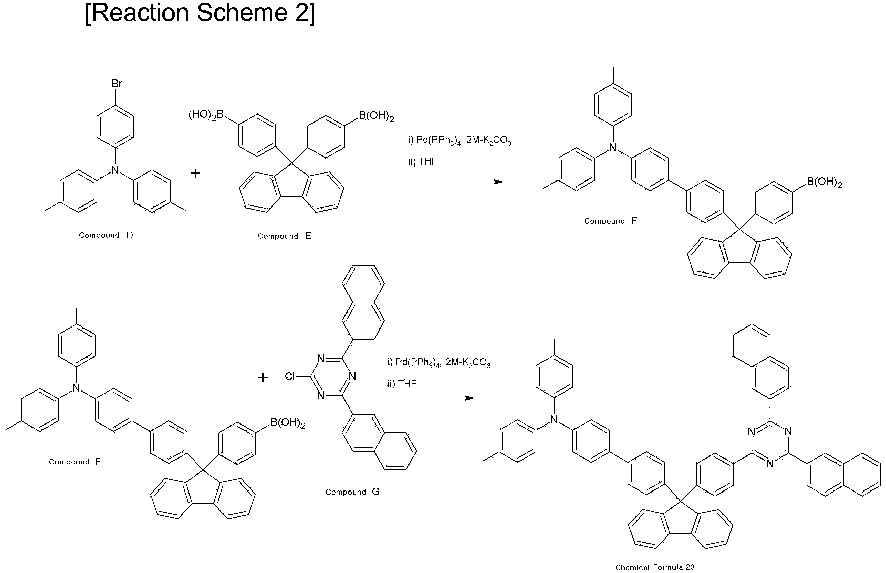

### First Step: Synthesis of compound F

5 g (12.3 mmol) of a compound E, 4.34 g (12.3 mmol) of a compound D were mixed with100 mL of tetrahydrofuran in a 250 mL round flask. Next, 80 mL of a 2M-potassium carbonate aqueous solution and 0.28 g (0.2 mmol) of tetrakistriphenylphosphine palladium (0) were added to the mixture. The resulting mixture was heated under a nitrogen gas flow for 12 hours.

The liquid reactant was separated into two layers, and the organic layer was dried with anhydrous sodium sulfate. Then, the organic solvent was distillated and removed under a reduced pressure. The residue was purified through a silica gel chromatography, obtaining 3 g of a compound represented by Chemical Formula F (yield: 40%).

### Second Step: Synthesis of Chemical Formula 23

3 g (4.7 mmol) of the compound F synthesized in the first step and 2.1 g (5.7 mmol) of the compound G were mixed with 80 mL of tetrahydrofuran in a 250 mL round flask. Next, 60 mL of a 2M-potassium carbonate aqueous solution and 0.11 g (0.1 mmol) of tetrakistriphenylphosphine palladium (0) were added to the mixture. The resulting mixture was heated and refluxed under a nitrogen gas flow for 12 hours.

Then, the reactant was dried using anhydrous sodium sulfate to separate an organic layer, and the organic solvent therein was distillated and removed under a reduced pressure. The resulting reactant was purified through a silica gel chromatography, obtaining 3.1 g of a compound represented by Chemical Formula 23 (yield: 70%).

The obtained compound represented by Chemical Formula 23 was performed regarding atomic analysis. The result is provided as follows;
calcd. C₅₂H₃₆N₄: C, 88.67; H, 5.25; N, 6.08; found: C, 88.52; H, 5.01; N, 6.03.

### Example 3: Synthesis of Compound represented by Chemical Formula 29

A compound represented by the above Chemical Formula 29 as a specific example of a compound for an organic photoelectric device according to the present invention was synthesized according to the following Reaction Scheme 3.

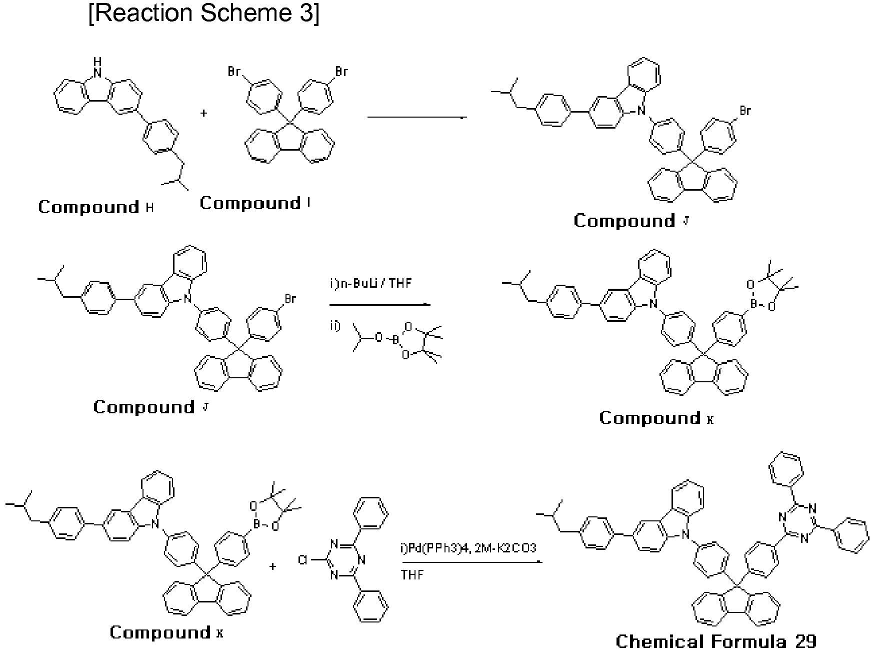

### First Step: Synthesis of Compound J

6.36 g (13.4 mmol) of the compound I, 4 g (13.4 mmol) of the compound H, 0.66 g (6.7 mmol) of copper chloride, and 5.5 g (40.1 mmol) of potassium carbonate were mixed with 100 mL of dimethylsulfoxide (DMSO) in a 250 mL round flask. A reactor was heated and agitated under a nitrogen gas flow at 180 ° C for 24 hours. When the reaction was complete, the solvent was vacuum-distillated and removed. The residue was purified through a silica gel chromatography, obtaining 5.9 g of a compound J represented by Chemical Formula J (yield: 64%).

### Second Step: Synthesis of Compound K

5 g (7.2 mmol) of the compound J was mixed with 150 mL of tetrahydrofuran in a 250 mL round-bottomed flask with an agitator under a nitrogen atmosphere and cooled down using dry ice and acetone. Next, 5.4 mL (8.64 mmol) of 1.6 M n-butyllithium (n-BuLi) was slowly injected to the reactor, and the dry ice for cooling was agitated for 30 minutes. Then, the reactor was cooled down, and 2 g (10.8 mmol) of 2-isopropoxy-4,4,5,5-tetramethyl-1,3,2-dioxaborane was slowly injected to the reactor. The reactor was agitated at a room temperature for 8 hours. The resulting reactant was poured into water to complete the reaction. Then, the reactant was extracted with ethylacetate and treated with anhydrous magnesium sulfate to remove moisture and a solvent therein. The residue was purified through a silica gel chromatography, obtaining 4 g of an intermediate product represented by a compound B (yield: 75 %).

### Third Step: Synthesis of Chemical Formula 29

4 g (5.39 mmol) of the compound K prepared in the second step and 1.73 g (6.5 mmol) of the compound C were synthesized with 80 mL of tetrahydrofuran in a 250 mL round-bottomed flask. Next, 60 mL of a 2M-potassium carbonate aqueous solution and 0.12 g (0.1 mmol) of tetrakistriphenylphosphine palladium (0) were added to the mixture. The resulting mixture was heated and refluxed under a nitrogen gas flow for 12 hours.

Then, an organic layer was separated from the reactant and dried with anhydrous sodium sulfate, and then, the organic solvent was distillated and removed under a reduced pressure. The resulting reactant was purified through a silica gel chromatography, obtaining 3.21 g of a compound represented by Chemical Formula 29 (yield: 70%).

The compound represented by Chemical Formula 29 was performed regarding atomic analysis. The result is provided as follows;
calcd. C₅₂H₃₆N₄: C, 87.91; H, 5.47; N, 6.61; found: C, 87.52; H, 5.04;, 6.32.

### Experimental Example 1: Thermal property evaluation

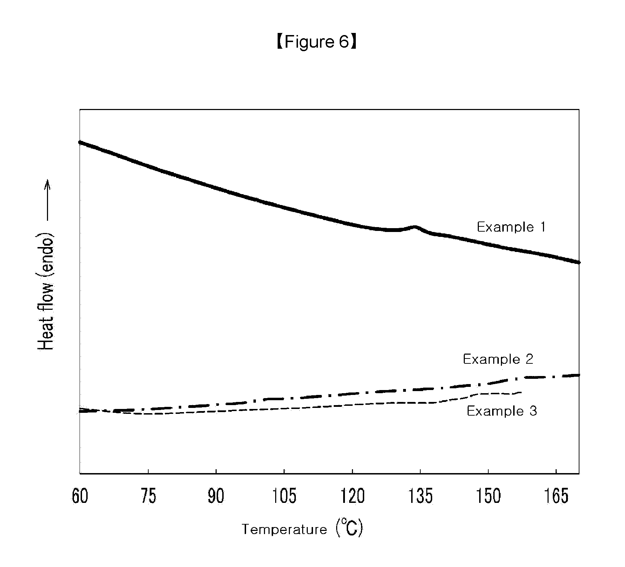

The compounds synthesized according to Examples 1 to 3 were measured regarding a glass transition temperature and a thermal decomposition temperature by respectively using a differential scanning calorimetry (DSC) and a thermogravimetry (TGA). The results are provided in the following Table 1 and FIG. 6.

**(Table 1)**

| | Glass transition temperature (° C) | Thermal decomposition temperature (° C) |
|---|---|---|
| Example 1 | 132 | 414 |
| Example 2 | 154 | 452 |
| Example 3 | 146 | 487 |

Referring to Table 1 and FIG. 6, the compounds according to Examples 1 to 3 had no melting point peak based on the result of the DSC analysis. The compounds according to Examples 1 to 3 were in a stable amorphous state. Accordingly, an organic photoelectric device including a compound for an organic photoelectric device according to one embodiment of the present invention was prevented from crystallization by Joule heat during the operation and would have improved life-span characteristic compared with a conventional organic photoelectric device.

### Preparation of Organic light emitting diode

### Example 4

An organic light emitting diode was fabricated by using the compound synthesized according to Example 1 as a host and Ir(PPy)₃ as a dopant. Herein, a 1000 A-thick ITO was used as an anode, while a 1000 A-thick aluminum (Al) was used as a cathode.

In particular, an ITO glass substrate having 15 Ω/cm² of sheet resistance was cut to have a size of 50 mm × 50 mm × 0.7 mm and respectively cleaned using ultrasonic wave in acetone, isopropyl alcohol, and pure water for 15 minutes and then, using UV ozone for 30 minutes, preparing an anode.

Next, 70 nm of N,N'-di(1-naphthyl)-N,N'-diphenylbenzidine (NPB) and 10 nm of 4,4',4"-tri(N-carbazoleyl)triphenylamine (TCTA) were deposited on the substrate with a vacuum degree of 650× 10⁻⁷ Pa at a deposition speed ranging from 0.1 to 0.3 nm/s to form a 800 A-thick hole transport layer (HTL).

Then, the compound synthesized according to Example 1 under the same vacuum-deposition condition was used to form a 300 A-thick emission layer. Herein, a phosphorescent dopant, Ir(PPy)₃, was simultaneously deposited. Herein, the phosphorescent dopant was regulated regarding deposition speed to be 7 wt% based on 100 wt% of the total weight of the emission layer.

On the emission layer, bis(8-hydroxy-2-methylquinolinolato)-aluminumbiphenoxide (BAlq) was deposited under the same vacuum deposition condition to form a 50 A-thick hole blocking layer.

Next, Alq₃ was deposited under the same vacuum deposition condition to form a 200 A-thick electron transport layer (ETL).

On the electron transport layer (ETL), LiF and Al were sequentially deposited to fabricate a cathode, fabricating an organic light emitting diode.

The organic light emitting diode had a structure of ITO/ NPB (70 nm)/ TCTA (10 nm)/ EML (30 nm, the compound of Example 1 (93 wt%) + Ir(PPy)₃ (7 wt%))/ Balq (5 nm)/ Alq (20 nm)/ LiF (1 nm) / Al (100 nm).

### Example 5

An organic light emitting diode was fabricated according to the same method as Example 4 except for forming no 50 A-thick hole blocking layer by depositing the BAlq.

In particular, the organic light emitting diode had a structure of ITO/ NPB (70 nm)/ TCTA (10nm)/ EML(30 nm, the compound (93 wt%) of Example 1 + Ir(PPy)₃ (7 wt%))/ Alq3 (20 nm)/ LiF (1 nm) / Al (100 nm).

### Example 6

An ITO substrate was used as an anode, and poly(3,4-ethylenedioxy-thiophene) (PEDOT) was spin-coated on the substrate.

On the PEDOT, and emission layer was formed in a spin-coating method. In particular, the emission layer was formed by dissolving the compound of Example 1 in toluene as a host and Ir(mppy)₃ as a dopant. Herein, the Ir(mppy)₃ dopant was mixed in an amount of 13 wt% based on 100 wt% of the total weight of the emission layer.

On the emission layer, BAlq was vacuum-deposited to form a 50 A-thick hole blocking layer. In addition, Alq₃ was vacuum-deposited to form a 200-thick electron transport layer (ETL) on the hole blocking layer.

On the electron transport layer (ETL), 10 A-thick (1 nm) LiF and 1000 A-thick Al were sequentially vacuum-deposited to fabricate a cathode, resultantly fabricating an organic light emitting diode.

The organic light emitting diode had a structure of ITO / PEDOT (40 nm) / EML (50 nm, the compound of Example 1 (87 wt%) + Ir(PPy)₃ (13 wt%)) / BAlq (5 nm) / Alq3 (20 nm) / LiF (1 nm) / Al (100 nm).

### Comparative Example 1

An organic light emitting diode was fabricated according to the same method as Example 4 except for using 4,4-N,N-dicarbazolebiphenyl (CBP) as a host for an emission layer instead of the compound synthesized in Example 1 as a host for an emission layer.

### Comparative Example 2

An organic light emitting diode was fabricated according to the same method as Example 6 except for using a mixture of 50 wt% of TCTA and 50 wt% of 1,3,5-tris(N-phenylbenzimidazole-2-yl)benzene (TPBI) instead of the compound synthesized in Example 1 as a host for an emission layer.

The organic light emitting diode had a structure of ITO / PEDOT (40 nm) / TCTA:TPBI (mixed in a weight ratio of 50:50, 87 wt%):Ir(mppy)₃ (13wt%) (50 nm) / BAlq (5 nm) / Alq₃ (20 nm) / LiF (1 nm) / Al (100 nm).

### Experimental Example 2: Performance Evaluation of Organic light emitting diode

Each organic light emitting diode according to Examples 4 to 6 and Comparative Examples 1 to 2 was measured regarding current density change and luminance change depending on voltage change and luminous efficiency. The measurement method was specifically as follows, and the results are provided in the following Tables 2 and 3.

### (1) Current density change depending on Voltage change

The organic light emitting diodes were measured regarding current using a current-voltage meter (Keithley 2400), while their voltages were increased from 0 V to 10 V. The current was divided by an area, obtaining current density.

### (2) Luminance change depending on Voltage change

The organic light emitting diodes were measured regarding current using a luminance meter (Minolta Cs-1000A), while their voltages were increased from 0 V to 10 V.

### (3) Luminous efficiency

The luminance and current density obtained from the (1) and (2) and voltage were used to calculate current efficiency (cd/A) and electric power efficiency (Im/W) at the same brightness (1000 cd/m²). The results were provided in the following Tables 2 and 3.

### (4) Color Coordinate

The organic light emitting diodes were measure regarding color coordinate by using a luminance meter (Minolta Cs-100A). The result is provided in the following Tables 2 and 3.

**(Table 2)**

| Device | Host material of emission layer | At 1000 cd/m² | | | |
|---|---|---|---|---|---|
| | | Driving voltage (V) | Current efficiency (cd/A) | Electric power efficiency (lm/W) | Color coordinate (x,y) |
| Example 4 | Example 1 | 6 | 48.88 | 25.59 | 0.30, 0.62 |
| Example 5 | Example 1 | 5.4 | 46.70 | 27.17 | 0.30, 0.62 |
| Comparative Example 1 | CBP | 7.8 | 46.80 | 18.85 | 0.29, 0.62 |

Referring to Table 2, the organic light emitting diodes according to Examples 4 and 5 had about more than 2 V lower driving voltage than the organic light emitting diode according to Comparative Example 1 but similar current efficiency to that of the organic light emitting diode according to Comparative Example 1. However, the organic light emitting diodes according to Examples 4 and 5 had much improved electric power efficiency in terms of performance due to decreased driving voltage. Particularly, the organic light emitting diode including no BAlq as a hole blocking layer according to Example 5 had very high luminous efficiency. Accordingly, a compound according to one embodiment of the present invention was found to have an advantage of decreasing cost and time in the manufacturing process due to decrease of a number of an organic thin layer.

**(Table 3)**

| Device | Host material of emission layer | At 1000 cd/m² | | | |
|---|---|---|---|---|---|
| | | Driving voltage (V) | Current efficiency (cd/A) | Electric power efficiency (lm/W) | Color coordinate (x,y) |
| Example 6 | Example 1 | 6.8 | 25.4 | 11.7 | 0.32, 0.62 |
| Comparative Example 2 | TCTA:TPBI | 7.51 | 14.5 | 6.13 | 0.31, 0.62 |

Referring to Table 3, the organic light emitting diode according to Example 6 had twice as much luminous efficiency as the one according to Comparative Example 2. Since TCTA was a host with excellent hole-transporting characteristic, and TPBI was a host with electron-transporting characteristic, the organic light emitting diode using the compound of Example 1 had excellent bipolar characteristic compared with the organic light emitting diode using a mixture of two compounds according to Comparative Example 2

## Claims

1. A compound for an organic photoelectric device represented by the following Chemical Formula 1: 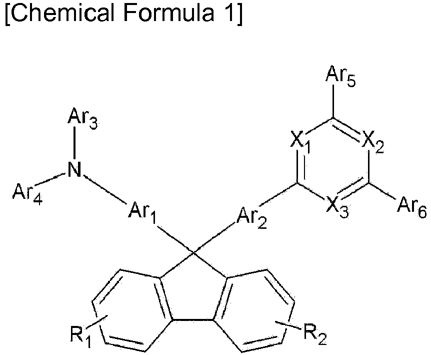 wherein, in Chemical Formula 1,
X₁ to X₃ are the same or different, and are each independently N or CR, provided that at least one of X₁ to X₃ is N, and R is hydrogen or a C1 to C10 lower alkyl group,
Ar₁ and Ar₂ are the same or different, and are each independently substituted or unsubstituted C6 to C18 arylene group, wherein the substituent of Ar₁ and Ar₂ is selected the group consisting of a C1 to C30 alkyl group, a C1 to C10 alkylsilyl group, a C3 to C30 cycloalkyl group, a C6 to C30 aryl group, a C1 to C10 alkoxy group, a fluoro group, a C1 to C10 trifluoroalkyl group, and a cyano,
Ar₃ and Ar₄ are the same or different, and are each independently substituted or unsubstituted C1 to C30 alkyl group, a substituted or unsubstituted C1 to C30 alkylene group, a substituted or unsubstituted C6 to C30 aryl group, a substituted or unsubstituted C6 to C30 arylene group, a substituted or unsubstituted C2 to C30 heteroaryl group, a substituted or unsubstituted C2 to C30 heteroarylene group, or a combination thereof, the Ar₃ and Ar₄ are fused to each other to provide a fused ring, wherein the substituent of Ar₃ and Ar₄ is selected the group consisting of a C1 to C30 alkyl group, a C1 to C10 alkylsilyl group, a C3 to C30 cycloalkyl group, a C6 to C30 aryl group, a C1 to C10 alkoxy group, a fluoro group, a C1 to C10 trifluoroalkyl group, and a cyano,
Ar₅ and Ar₆ are the same or different, and are each independently substituted or unsubstituted C1 to C30 alkyl group, a substituted or unsubstituted C6 to C30 aryl group, a substituted or unsubstituted C2 to C30 heteroaryl group, or a combination thereof, wherein the substituent of Ar₅ and Ar₆ is selected the group consisting of a C1 to C30 alkyl group, a C1 to C10 alkylsilyl group, a C3 to C30 cycloalkyl group, a C6 to C30 aryl group, a C1 to C10 alkoxy group, a fluoro group, a C1 to C10 trifluoroalkyl group, and a cyano, and
R₁ and R₂ are the same or different, and are each independently hydrogen or a C1 to C10 lower alkyl group.

2. The compound for an organic photoelectric device of claim 1, wherein all of the X₁ to X₃ of the above Chemical Formula 1 are N.

3. The compound for an organic photoelectric device of claim 1 or 2, wherein the arylene group of Ar₁ and Ar₂ in Chemical Formula 1 is a phenylene group, a naphthylene group, an anthracenylene group, or a combination thereof.

4. The compound for an organic photoelectric device of claims 1 to 3, wherein the aryl group of Ar₃ to Ar₆ of Chemical Formula 1 is a phenyl group, a naphthyl group, an anthracene group, a phenanthrene group, a tetracene group, a pyrene group, a fluorene group, or a combination thereof.

5. The compound for an organic photoelectric device of claims 1 to 4, wherein the aryl group of Ar₃ to Ar₆ of Chemical Formula 1 is a substituent represented by the following Chemical Formulas 2a to 2c, or a combination thereof: 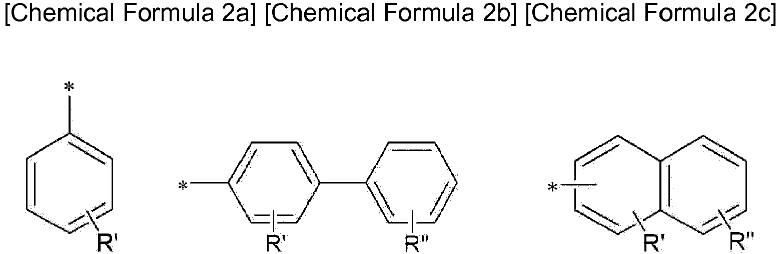 wherein, in Chemical Formulas 2a to 2c,
R' and R" are the same or different, and are each independently hydrogen, a C1 to C10 lower alkyl group, a C6 to C18 aryl group, or a combination thereof.

6. The compound for an organic photoelectric device of claims 1 to 5, wherein the heteroaryl group of Ar₅ and Ar₆ in Chemical Formula 1 is furan, pyrrole, imidazole, thiazole, oxazole, oxadiazole, thiadiazole, triazole, triazine, pyridine, pyrimidine, pyridazine, pyrazine, quinoline, isoquinoline, or a combination thereof.

7. The compound for an organic photoelectric device of claims 1 to 6, wherein the compound for an organic photoelectric device is represented by the following Chemical Formulas 3a or 3b: 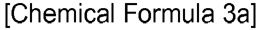 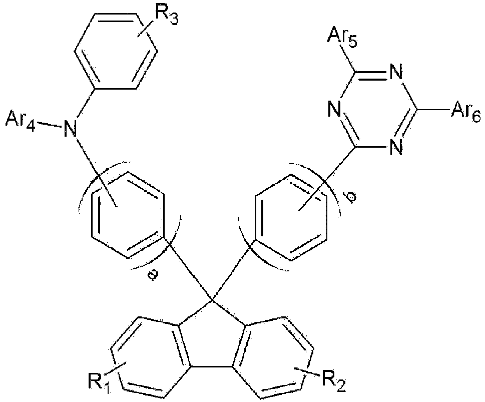 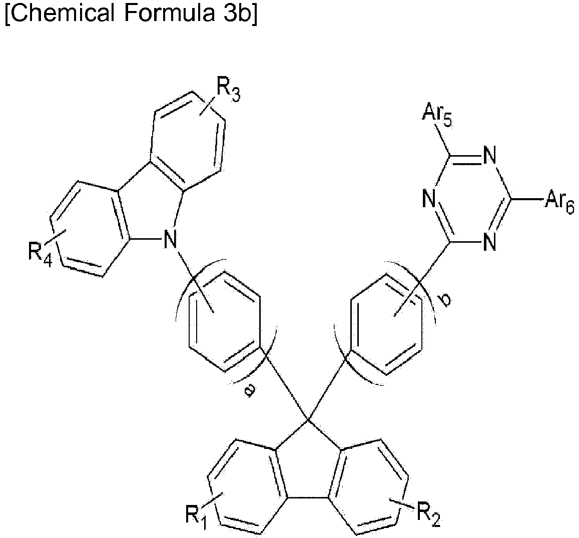 wherein, in Chemical Formulas 3a and 3b,
Ar₄ to Ar₆ are the same or different, and are each independently substituted or unsubstituted C1 to C30 alkyl group, a substituted or unsubstituted C6 to C30 aryl group, a substituted or unsubstituted C2 to C30 heteroaryl group, or a combination thereof,
R₁ and R₂ are the same or different, and are each independently hydrogen or a C1 to C10 lower alkyl group,
R₃ and R₄ are the same or different, and are each independently hydrogen, a C1 to C10 lower alkyl group, a C6 to C18 aryl group, or a combination thereof, and
a and b are the same or different, and are each independently 1 or 2.

8. The compound for an organic photoelectric device of claims 1 to 7, wherein the compound for an organic photoelectric device is represented by the following Chemical Formulas 4 to 35 and ad-1 to ad-4:

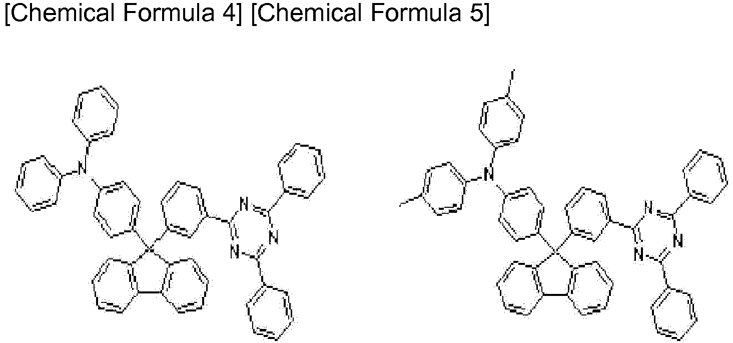

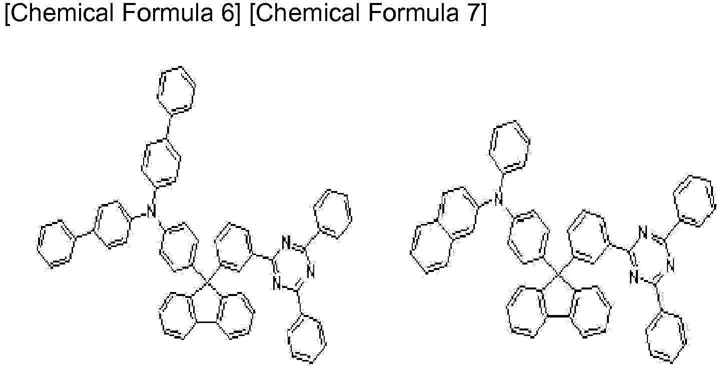

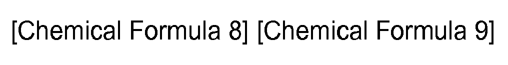

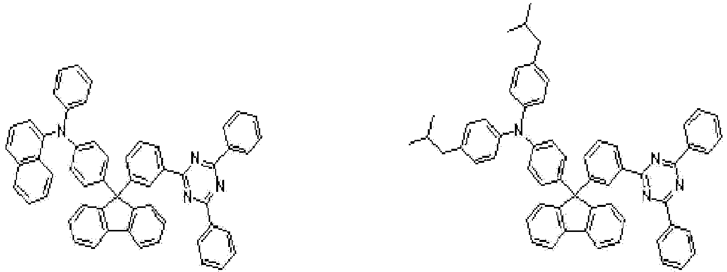

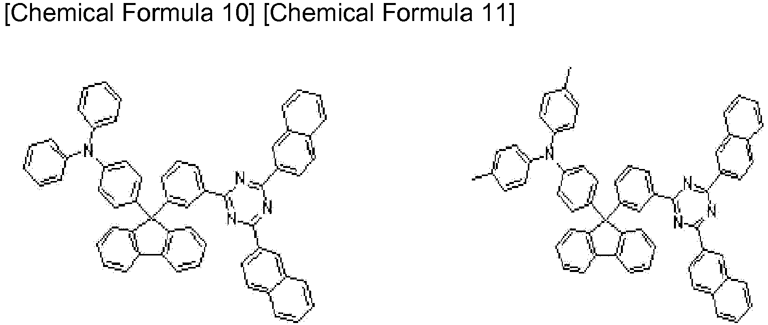

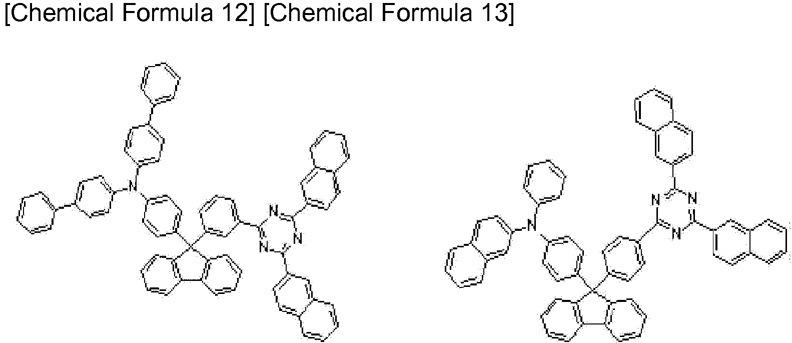

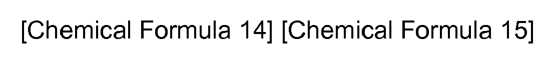

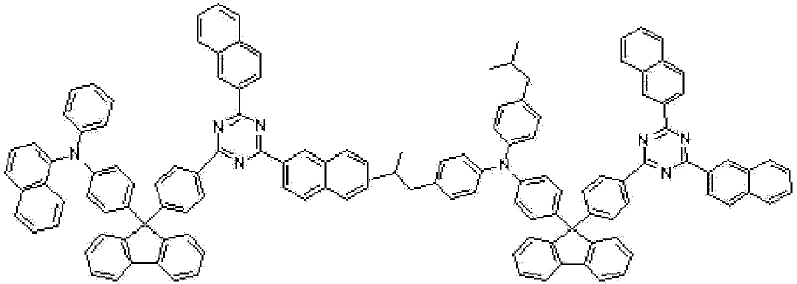

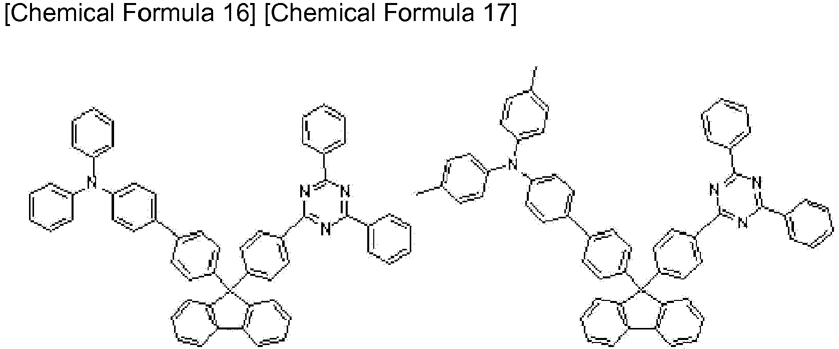

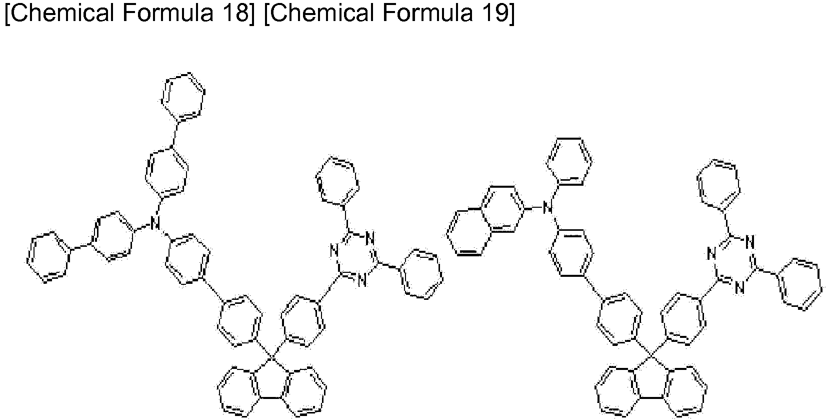

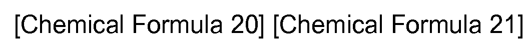

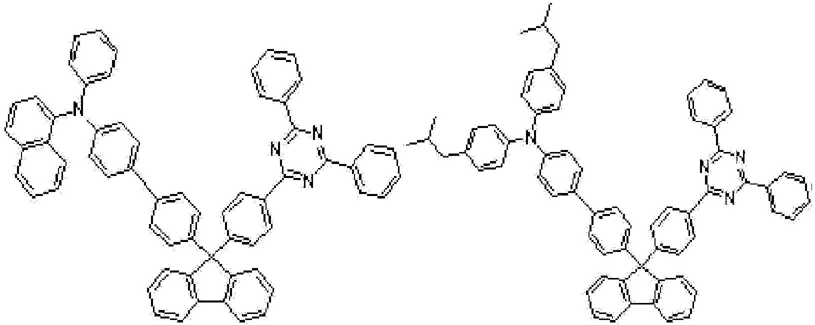

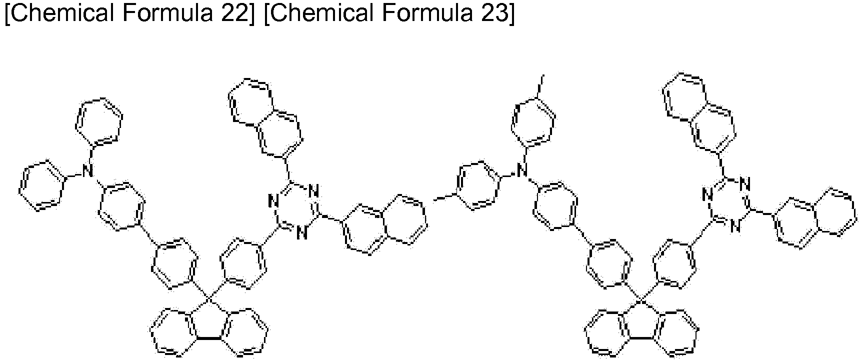

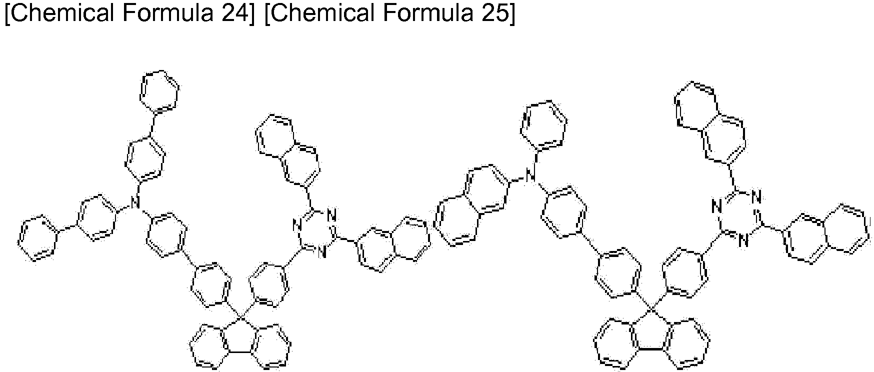

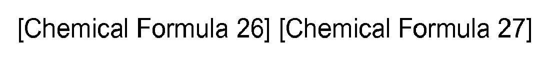

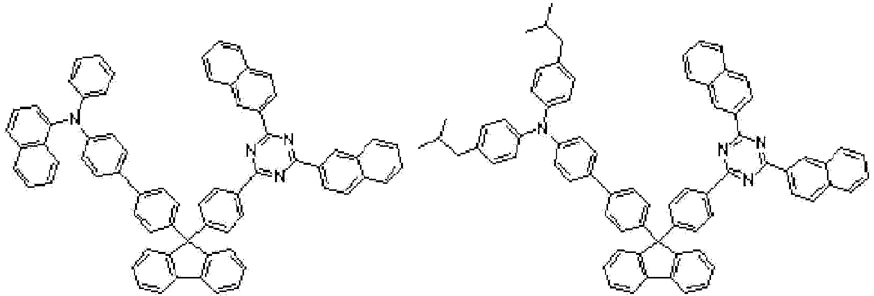

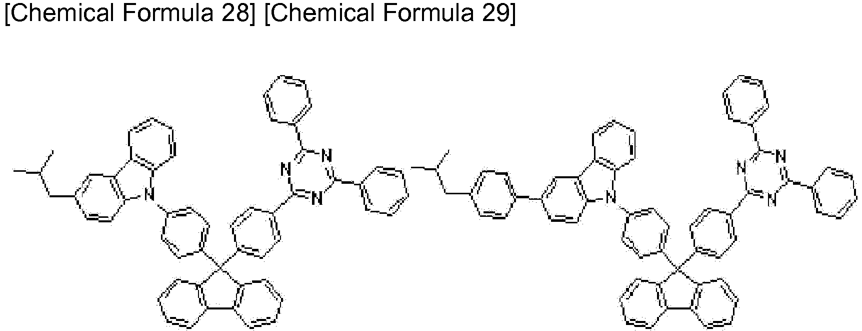

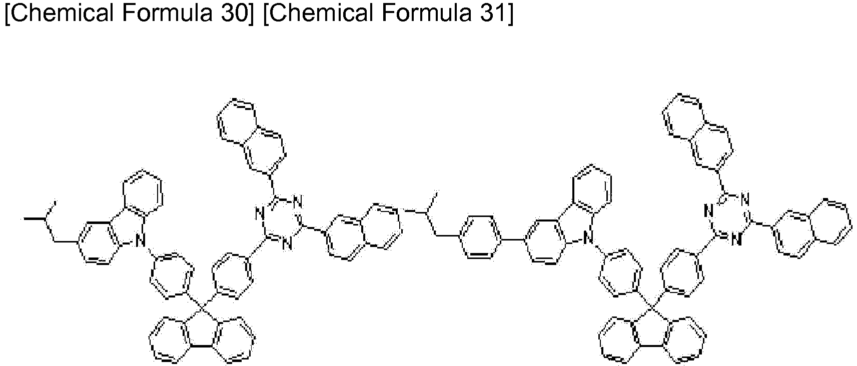

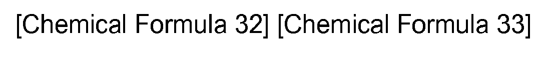

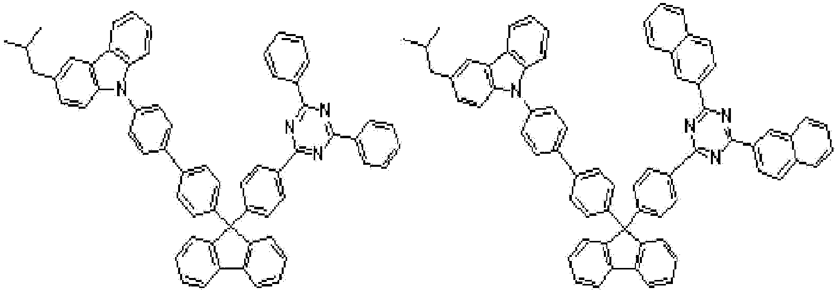

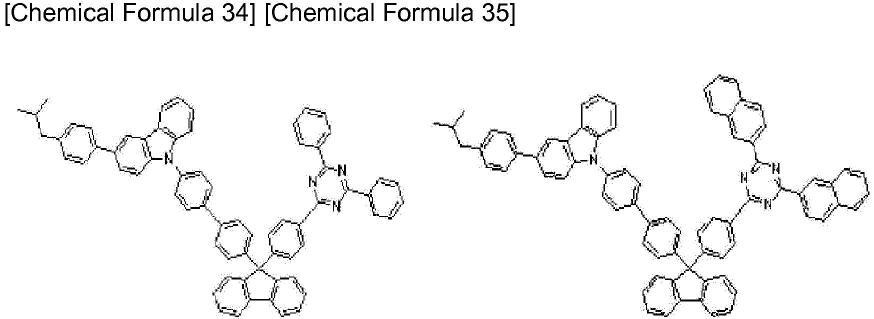

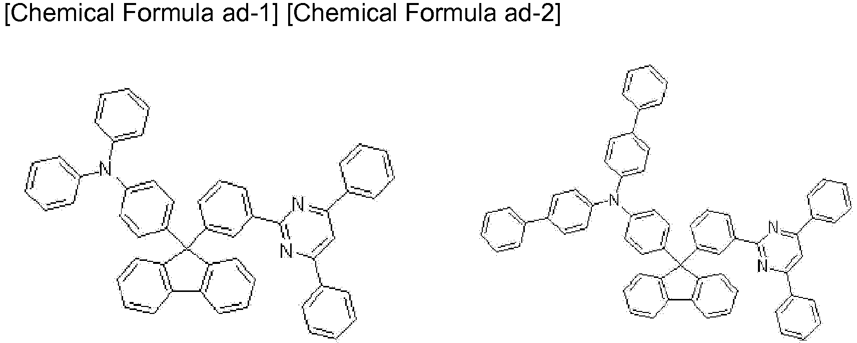

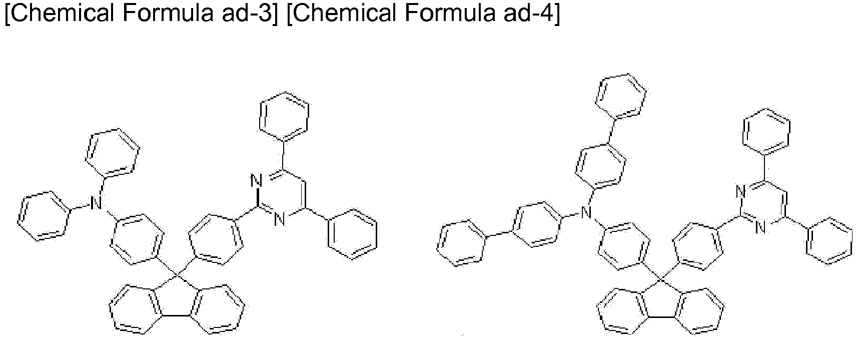

9. An organic photoelectric device comprising
an anode, a cathode and an organic thin layer between the anode and the cathode,
wherein the organic thin layer comprises the compound for an organic photoelectric device of any one of claim 1 to claim 8.

## Patentansprüche

1. Verbindung für eine organische photoelektrische Vorrichtung, die durch die folgende Chemische Formel 1 wiedergegeben wird: 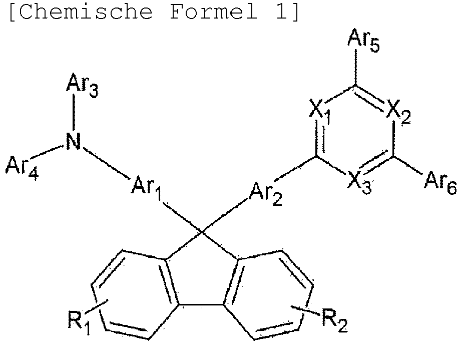 wobei in der Chemischen Formel 1
X₁ bis X₃ gleich oder verschieden sind und jeweils unabhängig für N oder CR stehen, mit der Maßgabe, dass mindestens eine der Variablen X₁ bis X₃ für N steht, und R für Wasserstoff oder eine C1- bis C10-Niederalkylgruppe steht,
Ar₁ und Ar₂ gleich oder verschieden sind und jeweils unabhängig für eine substituierte oder unsubstituierte C6- bis C18-Arylengruppe stehen, wobei der Substituent von Ar₁ und Ar₂ aus der Gruppe bestehend aus einer C1- bis C30-Alkylgruppe, einer C1- bis C10-Alkylsilylgruppe, einer C3- bis C30-Cycloalkylgruppe, einer C6- bis C30-Arylgruppe, einer C1- bis C10-Alkoxygruppe, einer Fluorgruppe, einer C1- bis C10-Trifluor-alkylgruppe und Cyano ausgewählt ist,
Ar₃ und Ar₄ gleich oder verschieden sind und jeweils unabhängig für eine substituierte oder unsubstituierte C1- bis C30-Alkylgruppe, eine substituierte oder unsubstituierte C1- bis C30-Alkylengruppe, eine substituierte oder unsubstituierte C6- bis C30-Arylgruppe, eine substituierte oder unsubstituierte C6- bis C30-Arylengruppe, eine substituierte oder unsubstituierte C2- bis C30-Heteroarylgruppe, eine substituierte oder unsubstituierte C2- bis C30-Heteroarylengruppe oder eine Kombination davon stehen oder Ar₃ und Ar₄ miteinander zu einem anellierten Ring anelliert sind, wobei der Substituent von Ar₃ und Ar₄ aus der Gruppe bestehend aus einer C1- bis C30-Alkylgruppe, einer C1- bis C10-Alkylsilylgruppe, einer C3- bis C30-Cycloalkylgruppe, einer C6- bis C30-Arylgruppe, einer C1-bis C10-Alkoxygruppe, einer Fluorgruppe, einer C1-bis C10-Trifluoralkylgruppe und Cyano ausgewählt ist,
Ar₅ und Ar₆ gleich oder verschieden sind und jeweils unabhängig für eine substituierte oder unsubstituierte C1- bis C30-Alkylgruppe, eine substituierte oder unsubstituierte C6- bis C30-Arylgruppe, eine substituierte oder unsubstituierte C2- bis C30-Heteroarylgruppe oder eine Kombination davon stehen, wobei der Substituent von Ar₅ und Arg aus der Gruppe bestehend aus einer C1- bis C30-Alkylgruppe, einer C1- bis C10-Alkylsilylgruppe, einer C3- bis C30-Cycloalkylgruppe, einer C6- bis C30-Arylgruppe, einer C1-bis C10-Alkoxygruppe, einer Fluorgruppe, einer C1-bis C10-Trifluoralkylgruppe und Cyano ausgewählt ist, und
R₁ und R₂ gleich oder verschieden sind und jeweils unabhängig für Wasserstoff oder eine C1- bis C10-Niederalkylgruppe stehen.

2. Verbindung für eine organische photoelektrische Vorrichtung nach Anspruch 1, wobei alle der Variablen X₁ bis X₃ der obigen Chemischen Formel 1 für N stehen.

3. Verbindung für eine organische photoelektrische Vorrichtung nach Anspruch 1 oder 2, wobei es sich bei der Arylengruppe von Ar₁ und Ar₂ in der Chemischen Formel 1 um eine Phenylengruppe, eine Naphthylengruppe, eine Anthracenylengruppe oder eine Kombination davon handelt.

4. Verbindung für eine organische photoelektrische Vorrichtung nach den Ansprüchen 1 bis 3, wobei es sich bei der Arylgruppe von Ar₃ bis Ar₆ der Chemischen Formel 1 um eine Phenylgruppe, eine Naphthylgruppe, eine Anthracengruppe, eine Phenanthrengruppe, eine Tetracengruppe, eine Pyrengruppe, eine Fluorengruppe oder eine Kombination davon handelt.

5. Verbindung für eine organische photoelektrische Vorrichtung nach den Ansprüchen 1 bis 4, wobei es sich bei der Arylgruppe von Ar₃ bis Ar₆ der Chemischen Formel 1 um einen Substituenten, der durch die folgenden Chemischen Formeln 2a bis 2c wiedergegeben wird, oder eine Kombination davon handelt: 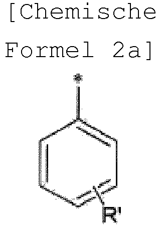 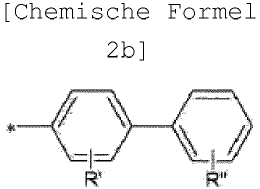 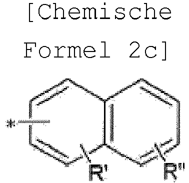 wobei in den Chemischen Formeln 2a bis 2c
R' und R'' gleich oder verschieden sind und jeweils unabhängig für Wasserstoff, eine C1- bis C10-Niederalkylgruppe, eine C6- bis C18-Arylgruppe oder eine Kombination davon stehen.

6. Verbindung für eine organische photoelektrische Vorrichtung nach den Ansprüchen 1 bis 5, wobei es sich bei der Heteroarylgruppe von Ar₅ bis Ar₆ in der Chemischen Formel 1 um Furan, Pyrrol, Imidazol, Thiazol, Oxazol, Oxadiazol, Thiadiazol, Triazol, Triazin, Pyridin, Pyrimidin, Pyridazin, Pyrazin, Chinolin, Isochinolin oder eine Kombination davon handelt.

7. Verbindung für eine organische photoelektrische Vorrichtung nach den Ansprüchen 1 bis 6, wobei die Verbindung für eine organische photoelektrische Vorrichtung durch die folgenden Chemischen Formeln 3a oder 3b wiedergegeben wird: 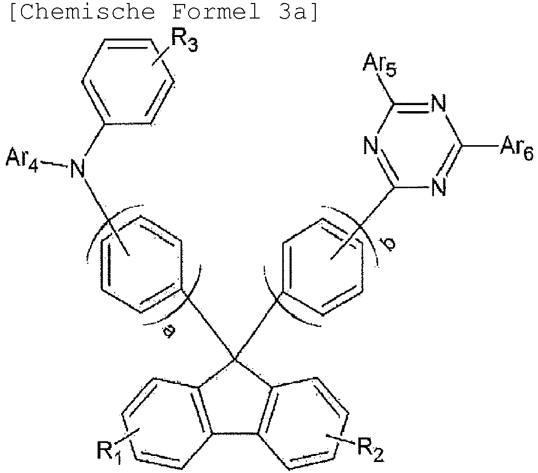 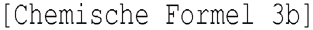 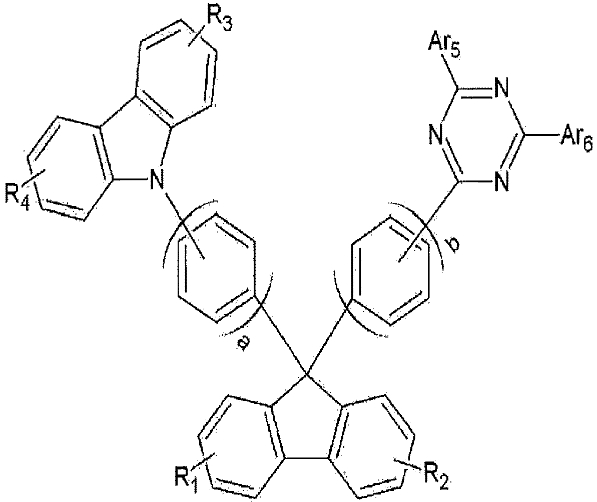 wobei in den Chemischen Formeln 3a und 3b
Ar₄ bis Ar₆ gleich oder verschieden sind und jeweils unabhängig für eine substituierte oder unsubstituierte C1- bis C30-Alkylgruppe, eine substituierte oder unsubstituierte C6- bis C30-Arylgruppe, eine substituierte oder unsubstituierte C2- bis C30-Heteroarylgruppe oder eine Kombination davon stehen,
R₁ und R₂ gleich oder verschieden sind und jeweils unabhängig für Wasserstoff oder eine C1- bis C10-Niederalkylgruppe stehen,
R₃ und R₄ gleich oder verschieden sind und jeweils unabhängig für Wasserstoff, eine C1- bis C10-Niederalkylgruppe, eine C6- bis C18-Arylgruppe oder eine Kombination davon stehen und
a und b gleich oder verschieden sind und jeweils unabhängig für 1 oder 2 stehen.

8. Verbindung für eine organische photoelektrische Vorrichtung nach den Ansprüchen 1 bis 7, wobei die Verbindung für eine organische photoelektrische Vorrichtung durch die folgenden Chemischen Formeln 4 bis 35 und ad-1 bis ad-4 wiedergegeben wird:

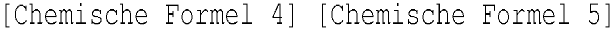

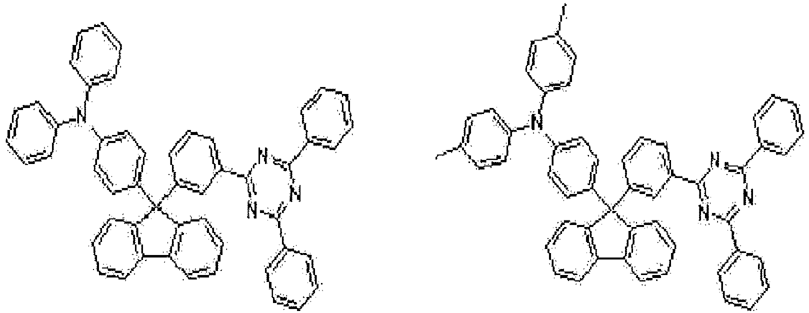

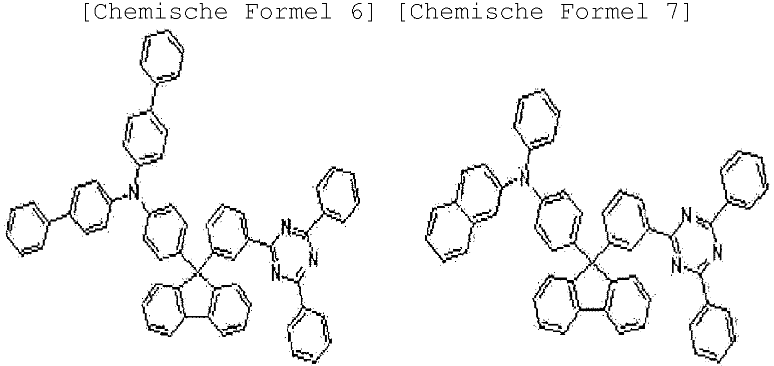

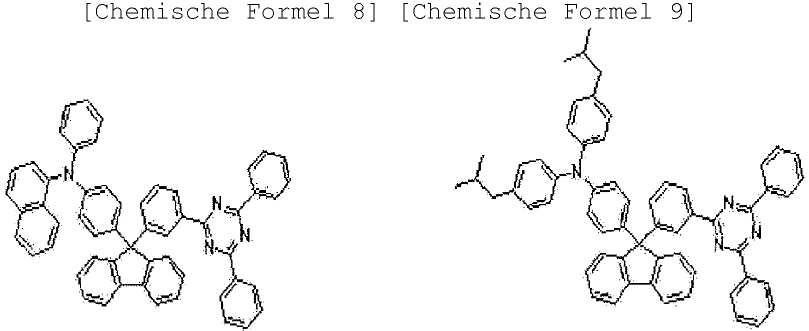

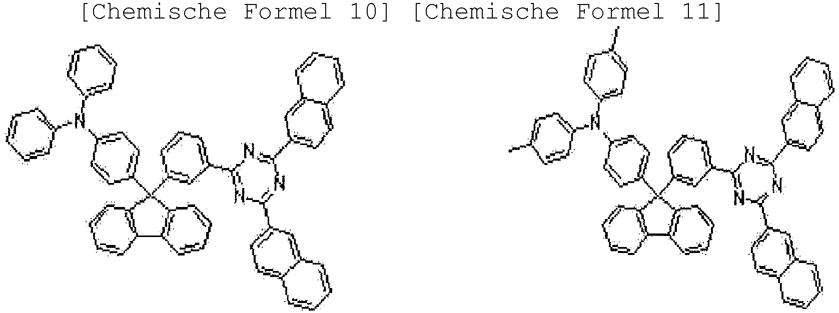

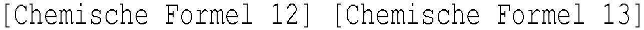

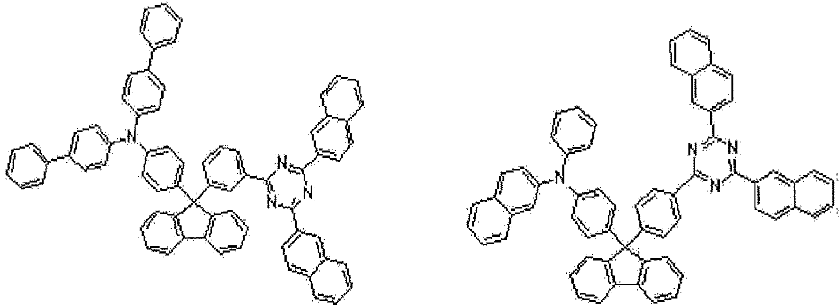

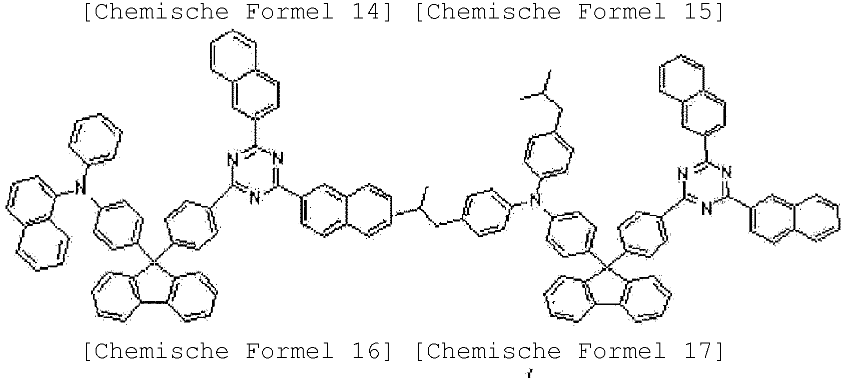

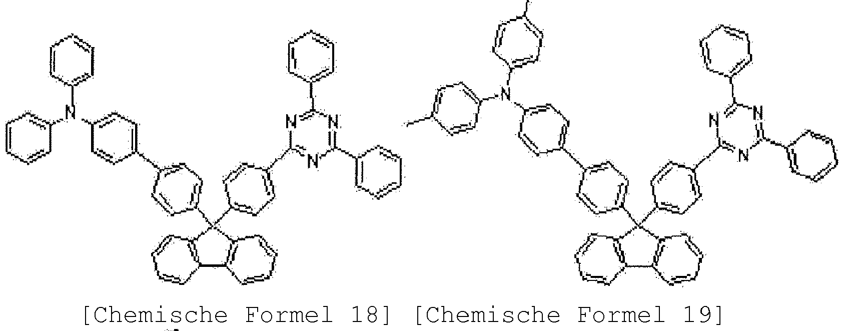

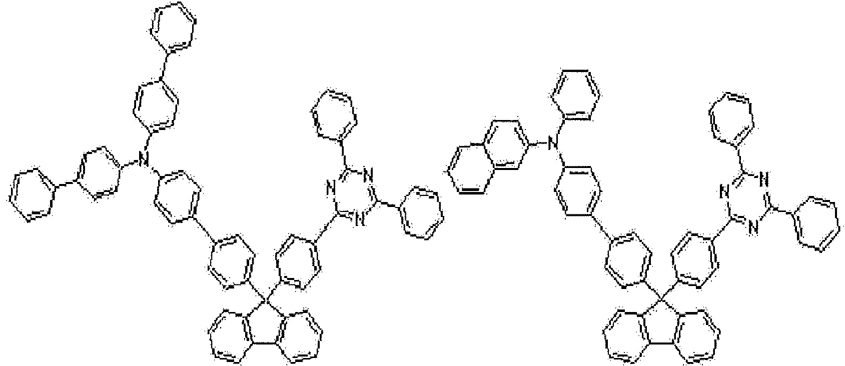

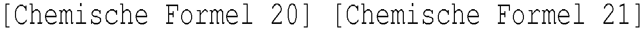

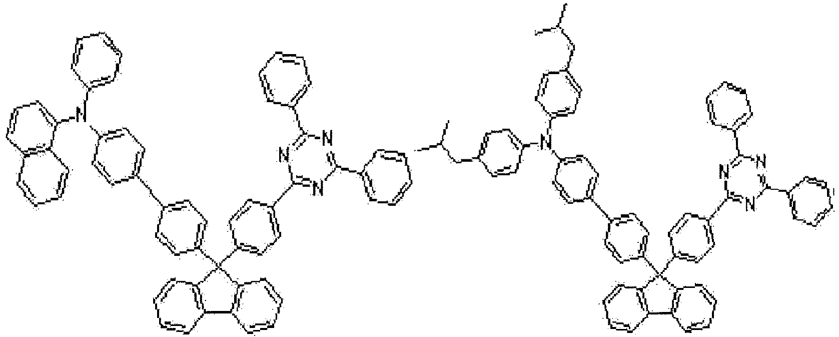

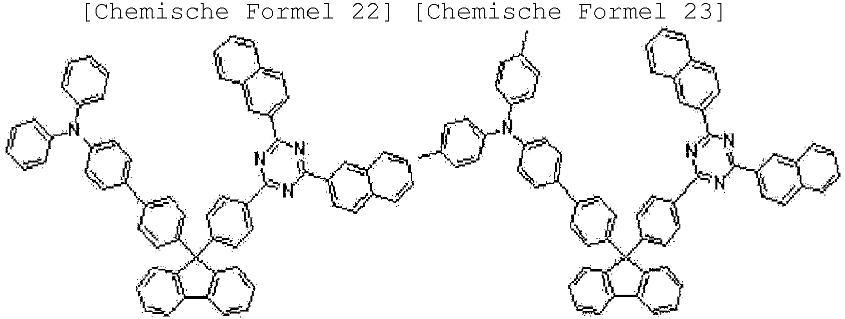

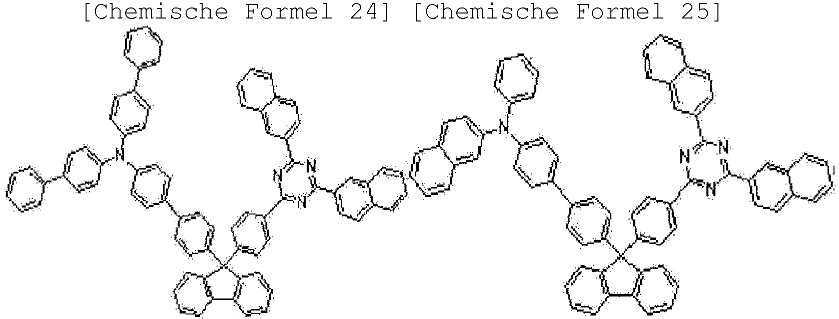

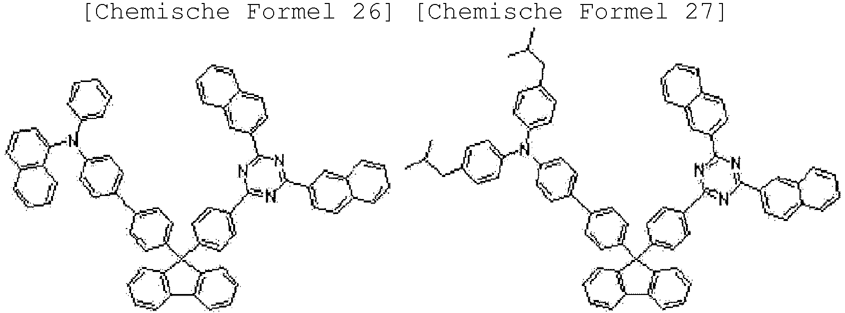

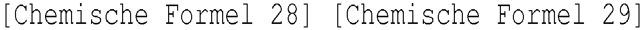

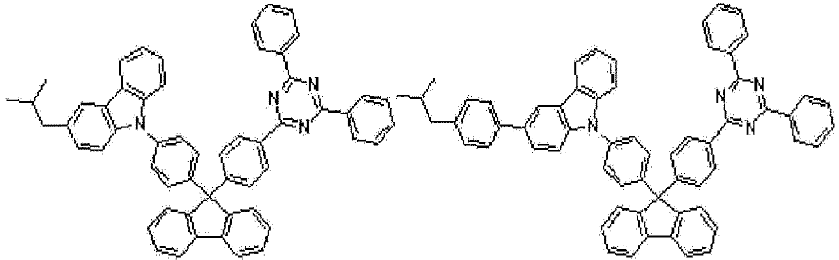

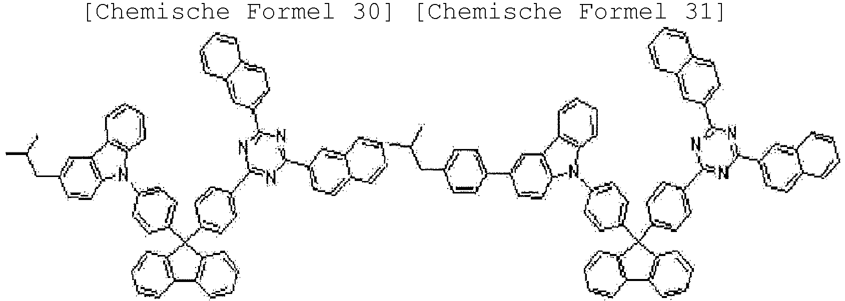

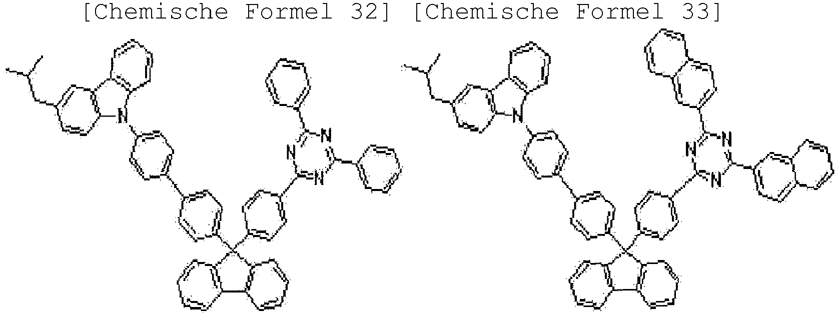

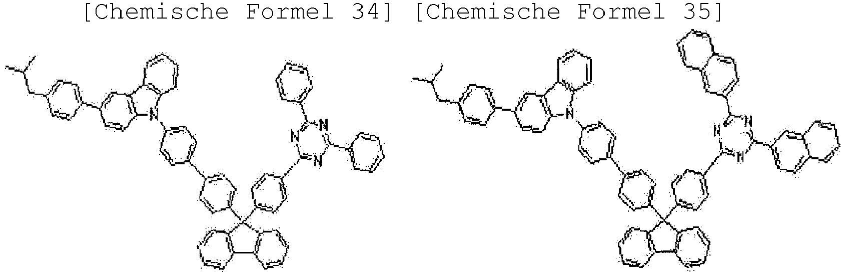

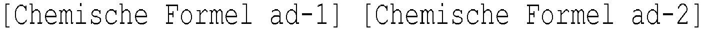

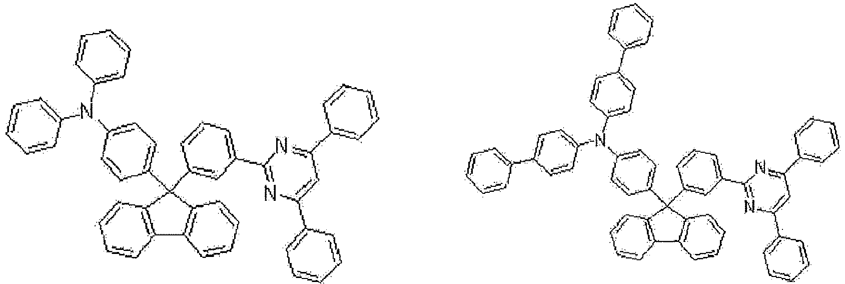

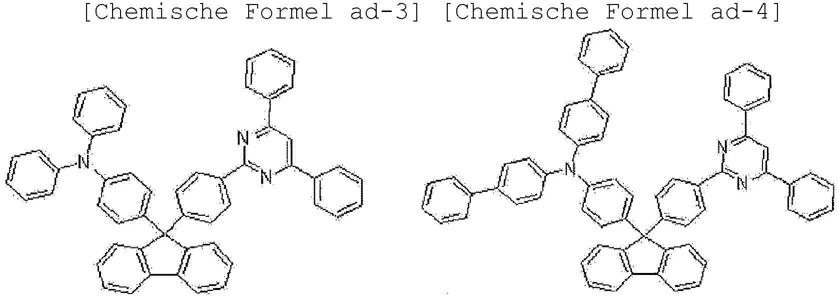

9. Organische photoelektrische Vorrichtung, umfassend eine Anode, eine Kathode und eine organische dünne Schicht zwischen der Anode und Kathode,
wobei die organische dünne Schicht die Verbindung für eine organische photoelektrische Vorrichtung nach einem der Ansprüche 1 bis 8 umfasst.

## Revendications

1. Composé pour un dispositif photoélectrique organique représenté par la formule chimique 1 suivante : 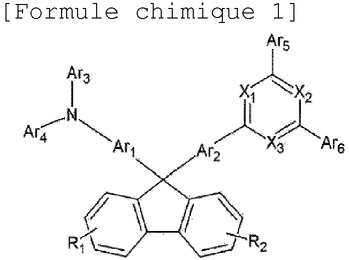 dans lequel, dans la formule chimique 1,
X₁ à X₃ sont identiques ou différents, et représentent chacun indépendamment N ou CR, à condition qu'au moins un de X₁ à X₃ représente N, et R représente un hydrogène ou un groupe alkyle inférieur en C₁ à C₁₀,
Ar₁ et Ar₂ sont identiques ou différents, et représentent chacun indépendamment un groupe arylène en C₆ à C₁₈ substitué ou non substitué, le substituant de Ar₁ et Ar₂ étant choisi dans le groupe constitué par un groupe alkyle en C₁ à C₃₀, un groupe alkylsilyle en C₁ à C₁₀, un groupe cycloalkyle en C₃ à C₃₀, un groupe aryle en C₆ à C₃₀, un groupe alcoxy en C₁ à C₁₀, un groupe fluoro, un groupe trifluoroalkyle en C₁ à C₁₀, et un groupe cyano,
Ar₃ et Ar₄ sont identiques ou différents, et représentent chacun indépendamment un groupe alkyle en C₁ à C₃₀ substitué ou non substitué, un groupe alkylène en C₁ à C₃₀ substitué ou non substitué, un groupe aryle en C₆ à C₃₀ substitué ou non substitué, un groupe arylène en C₆ à C₃₀ substitué ou non substitué, un groupe hétéroaryle en C₂ à C₃₀ substitué ou non substitué, un groupe hétéroarylène en C₂ à C₃₀ substitué ou non substitué, ou une combinaison de ceux-ci, Ar₃ et Ar₄ sont condensés l'un à l'autre pour former un cycle condensé, le substituant de Ar₃ et Ar₄ étant choisi dans le groupe constitué par un groupe alkyle en C₁ à C₃₀, un groupe alkylsilyle en C₁ à C₁₀, un groupe cycloalkyle en C₃ à C₃₀, un groupe aryle en C₆ à C₃₀, un groupe alcoxy en C₁ à C₁₀, un groupe fluoro, un groupe trifluoroalkyle en C₁ à C₁₀, et un groupe cyano,
Ar₅ et Ar₆ sont identiques ou différents, et représentent chacun indépendamment un groupe alkyle en C₁ à C₃₀ substitué ou non substitué, un groupe aryle en C₆ à C₃₀ substitué ou non substitué, un groupe hétéroaryle en C₂ à C₃₀ substitué ou non substitué, ou une combinaison de ceux-ci, le substituant de Ar₅ et Ar₆ étant choisi dans le groupe constitué par un groupe alkyle en C₁ à C₃₀, un groupe alkylsilyle en C₁ à C₁₀, un groupe cycloalkyle en C₃ à C₃₀, un groupe aryle en C₆ à C₃₀, un groupe alcoxy en C₁ à C₁₀, un groupe fluoro, un groupe trifluoroalkyle en C₁ à C₁₀, et un groupe cyano, et
R₁ et R₂ sont identiques ou différents, et représentent chacun indépendamment un hydrogène ou un groupe alkyle inférieur en C₁ à C₁₀.

2. Composé pour un dispositif photoélectrique organique de la revendication 1, dans lequel tous les X₁ à X₃ de la formule chimique 1 ci-dessus représentent N.

3. Composé pour un dispositif photoélectrique organique de la revendication 1 ou 2, dans lequel le groupe arylène de Ar₁ et Ar₂ dans la formule chimique 1 est un groupe phénylène, un groupe naphtylène, un groupe anthracénylène, ou une combinaison de ceux-ci.

4. Composé pour un dispositif photoélectrique organique des revendications 1 à 3, dans lequel le groupe aryle de Ar₃ à Ar₆ de la formule chimique 1 est un groupe phényle, un groupe naphtyle, un groupe anthracène, un groupe phénanthrène, un groupe tétracène, un groupe pyrène, un groupe fluorène, ou une combinaison de ceux-ci.

5. Composé pour un dispositif photoélectrique organique des revendications 1 à 4, dans lequel le groupe aryle de Ar₃ à Ar₆ de la formule chimique 1 est un substituant représenté par les formules chimiques 2a à 2c suivantes, ou une combinaison de ceux-ci : 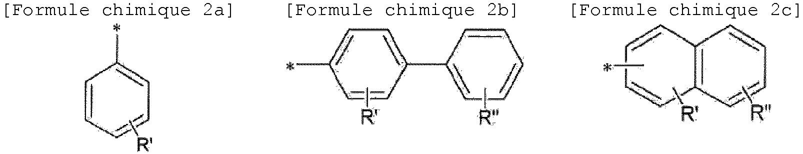 dans lequel, dans les formules chimiques 2a à 2c,
R' et R" sont identiques ou différents, et représentent chacun indépendamment un hydrogène, un groupe alkyle inférieur en C₁ à C₁₀, un groupe aryle en C₆ à C₁₈, ou une combinaison de ceux-ci.

6. Composé pour un dispositif photoélectrique organique des revendications 1 à 5, dans lequel le groupe hétéroaryle de Ar₅ et Ar₆ dans la formule chimique 1 est un groupe furane, pyrrole, imidazole, thiazole, oxazole, oxadiazole, thiadiazole, triazole, triazine, pyridine, pyrimidine, pyridazine, pyrazine, quinoline, isoquinoline, ou une combinaison de ceux-ci.

7. Composé pour un dispositif photoélectrique organique des revendications 1 à 6, le composé pour un dispositif photoélectrique organique étant représenté par les formules chimiques 3a ou 3b suivantes : 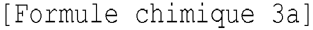 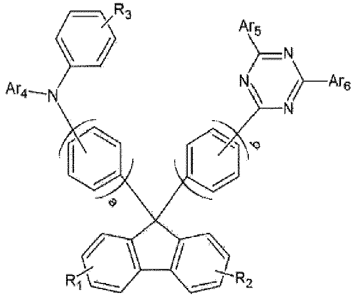 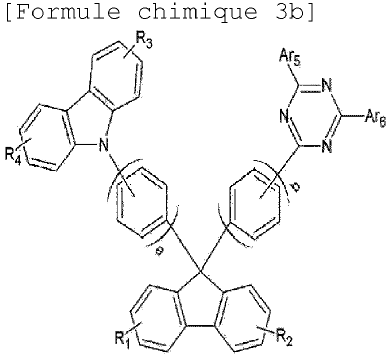 dans lequel, dans les formules chimiques 3a et 3b,
Ar₄ à Ar₆ sont identiques ou différents, et représentent chacun indépendamment un groupe alkyle en C₁ à C₃₀ substitué ou non substitué, un groupe aryle en C₆ à C₃₀ substitué ou non substitué, un groupe hétéroaryle en C₂ à C₃₀ substitué ou non substitué, ou une combinaison de ceux-ci,
R₁ et R₂ sont identiques ou différents, et représentent chacun indépendamment un hydrogène ou un groupe alkyle inférieur en C₁ à C₁₀,
R₃ et R₄ sont identiques ou différents, et représentent chacun indépendamment un hydrogène, un groupe alkyle inférieur en C₁ à C₁₀, un groupe aryle en C₆ à C₁₈, ou une combinaison de ceux-ci, et
a et b sont identiques ou différents, et valent chacun indépendamment 1 ou 2.

8. Composé pour un dispositif photoélectrique organique des revendications 1 à 7, le composé pour un dispositif photoélectrique organique étant représenté par les formules chimiques 4 à 35 et ad-1 à ad-4 suivantes :

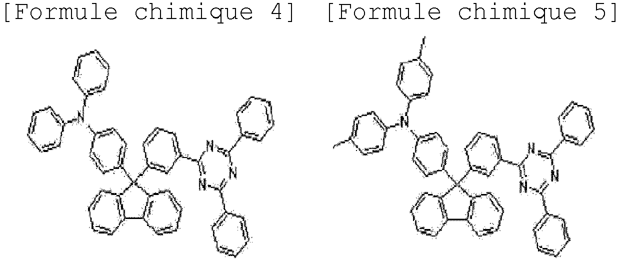

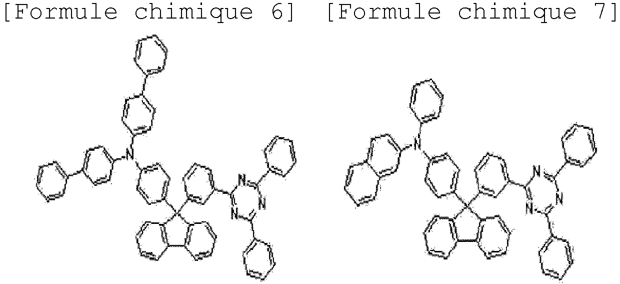

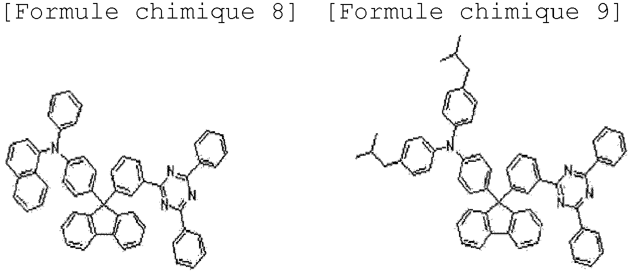

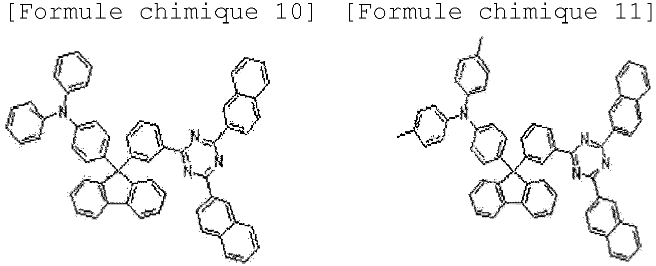

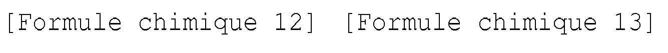

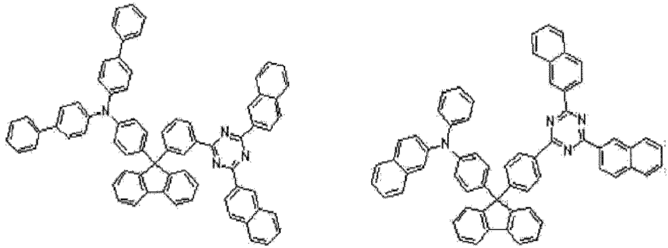

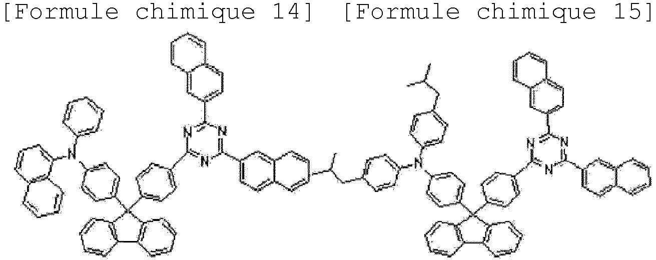

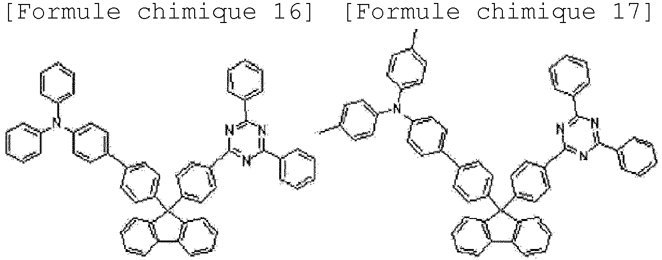

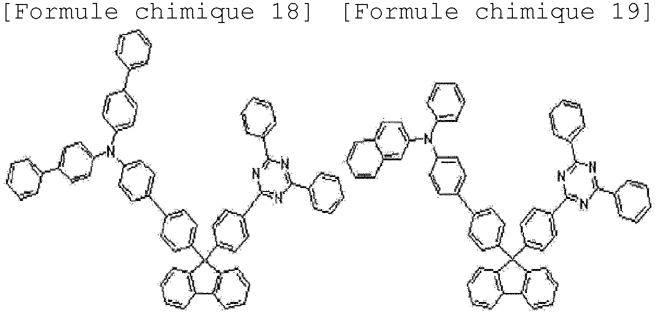

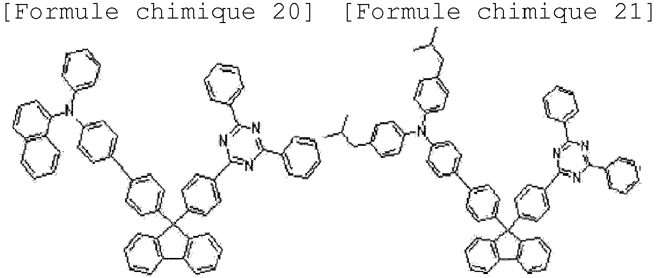

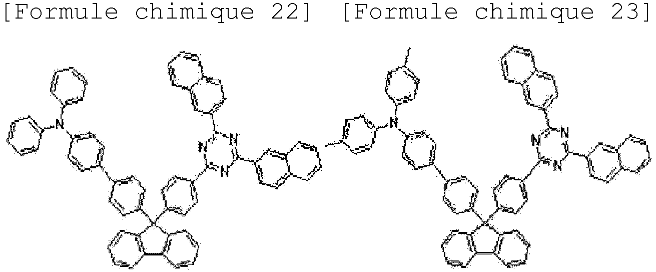

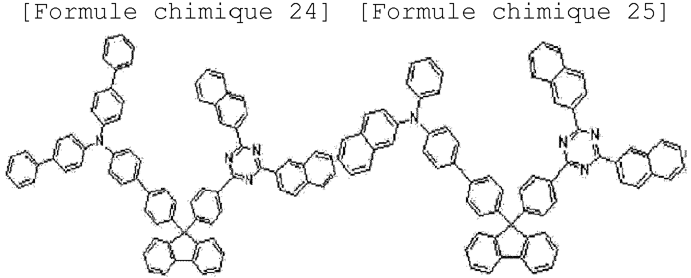

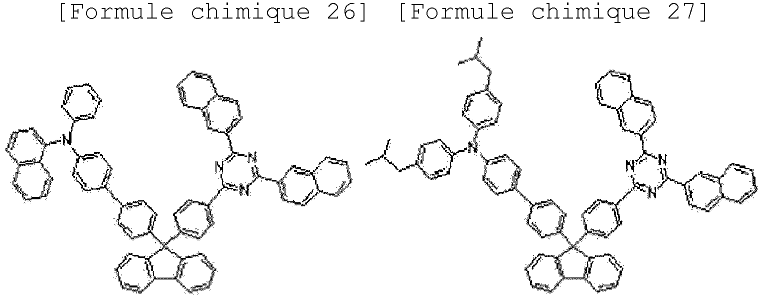

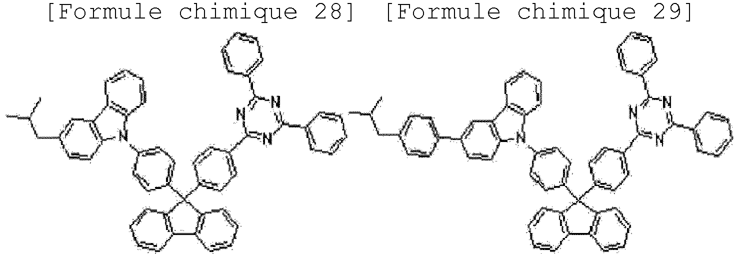

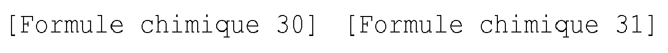

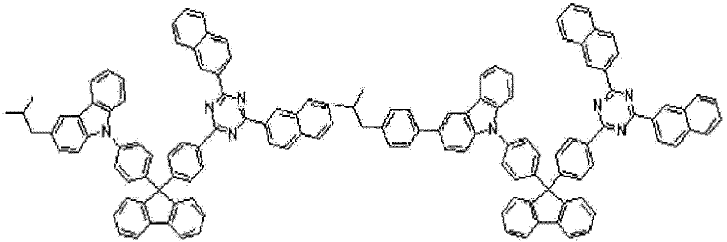

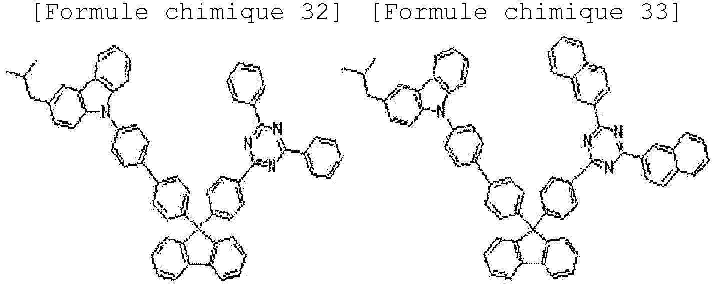

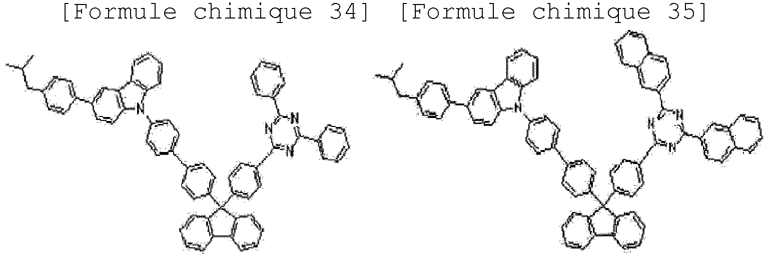

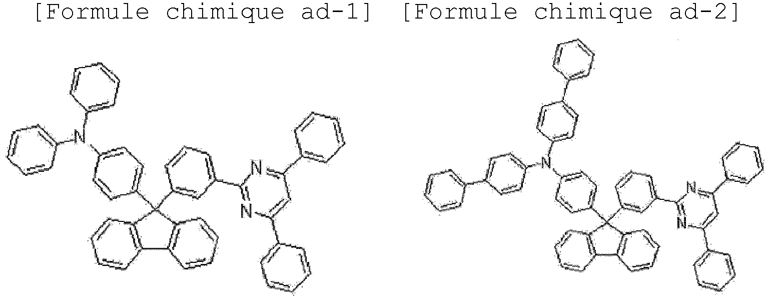

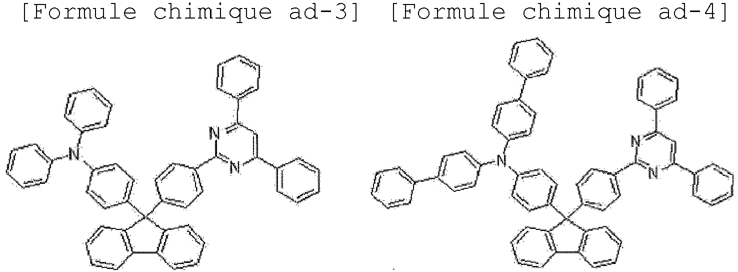

9. Dispositif photoélectrique organique comprenant
une anode, une cathode et une couche mince organique entre l'anode et la cathode,
la couche mince organique comprenant le composé pour un dispositif photoélectrique organique selon l'une quelconque des revendications 1 à 8.
